# EUROPÄISCHE PATENTSCHRIFT

(11) **EP 2 445 551 B1**
(45) Veröffentlichungstag und Bekanntmachung des Hinweises auf die Patenterteilung: **18.09.2019**
(21) Anmeldenummer: 09779912.6
(22) Anmeldetag: 23.06.2009
(51) Int. Cl.: A61M 5/315, A61M 5/20

(54) **INJEKTIONSVORRICHTUNG MIT EINEM DOSIERMECHANISMUS ZUM BEGRENZEN EINER DOSISEINSTELLUNG**
INJECTION DEVICE HAVING A DOSING MECHANISM FOR LIMITING A DOSE SETTING
DISPOSITIF D'INJECTION MUNI D'UN MÉCANISME DE DOSAGE POUR LIMITER UN RÉGLAGE DE DOSE

(43) Veröffentlichungstag der Anmeldung: 02.05.2012
(73) Patentinhaber: Tecpharma Licensing AG, 3401 Burgdorf (CH)
(72) Erfinder: FRANTZ, Markus, 85604 Pöring (DE); STETTLER, Peter, CH-3422 Kirchberg (CH); KLADIWA, Malte, CH-3008 Bern (CH); HOSTETTLER, Patrick, CH-3415 Hasle-Rüegsau (CH); MORI, Kevin, CH-3415 Hasle b. Burgdorf (CH); HORISBERGER, Aurèle, CH-4123 Allschwil (CH); WITTMANN, Jürgen, CH-3400 Burgdorf (CH)
(86) Internationale Anmeldenummer: PCT/EP2009/057854
(87) Internationale Veröffentlichungsnummer: WO 2010/149209

(56) Entgegenhaltungen:
- EP-A2- 1 944 050
- EP-A2- 2 011 531
- WO-A1-2007/017053
- WO-A1-2008/031238
- WO-A1-2009/105909
- DE-A1-102004 039 846
- US-A- 3 202 151

## Beschreibung

Die Erfindung betrifft einen Mechanismus für eine Injektionsvorrichtung der das Einstellen einer Dosis verhindert, welche die Menge eines zu verabreichenden Produkts in einem Produktbehältnis der Injektionsvorrichtung übersteigt. Bei dem zu verabreichenden Produkt kann es sich z.B. um ein flüssiges Medikament, insbesondere um Insulin handeln.

Aus dem Stand der Technik ist ein Dosiermechanismus für eine Injektionsvorrichtung zum Verhindern der Einstellung einer Dosis bekannt, welche die Medikamentenmenge in einem Reservoir einer Injektionsvorrichtung übersteigt. Solche Vorrichtungen haben oftmals das Problem, relativ viel Platz in der Injektionsvorrichtung zu verbrauchen. Andererseits verlangt der Markt nach handlichen Injektionsvorrichtungen.

Aus der WO2008/031238 A1 ist eine Vorrichtung bekannt, welche das Einstellen einer Dosis verhindert, welche die Medikamentenmenge in einem Reservoir übersteigt. Die Vorrichtung besteht dabei aus einem Dosisbegrenzungsring, welcher über ein Aussengewinde aufweist, das in Gewindeeingriff mit einem Innengewinde einer Kupplungshülse steht. Auf der Innenseite weist der Dosisbegrenzungsring axial orientierte Längsführungen auf, welche in entsprechende Gegenführungen auf einer Übertragungshülse angeordnet sind. Der Dosisbegrenzungsring ist also gegenüber der Übertragungshülse verdrehgesichert. Beim Einstellen einer Dosis rotiert die koaxial zur Übertragungshülse angeordnete Kupplungshülse relativ zur Übertragungshülse. Durch den Gewindeeingriff zwischen Kupplungshülse und Dosisbegrenzungsring verschiebt sich der Dosisbegrenzungsring in Richtung eines Anschlags, wobei der Gesamtweg, welcher der Dosisbegrenzungsring zurücklegen kann, der im Reservoir vorhandenen Menge an Medikament entspricht. Erreicht der Dosisbegrenzungsring das Ende des Gewindes an der Kupplungshülse, so wird eine weitere Relativdrehung zwischen Übertragungshülse und Kupplungshülse verhindert, wodurch eine weitere Erhöhung der Dosis nicht mehr möglich ist.

Aus der WO2007/017053 A1 ist eine analoge Vorrichtung zur Dosisbegrenzug bekannt, wobei der Dosisbegrenzungsring zwischen einem Treiber und einer Kolbenstange angeordnet ist sowie ein Innengewinde und auf seiner Aussenseite Längsführungen aufweist. Die Funktionsweise ist mit der in der WO2008/031238 vergleichbar.

Eine weitere Vorrichtung zur Dosisbegrenzung ist aus der EP1944050 A2 bekannt. Diese Vorrichtung funktioniert analog zur Vorrichtung aus WO2007/017053 A1, wobei der Dosisbegrenzungring zwischen einer Dosierhülse und einer Treiberhülse angeordnet ist.

Aus der EP2011531 A2 ist ebenfalls eine Vorrichtung zur Dosisbegrenzung bekannt. Im Unterschied zu den oben beschriebenen Beispielen ist das Dosisbegrenzungselement nicht als voller Ring ausgebildet, sondern nur als Ringsegment.

Alle vier beschriebenen Dokumente aus dem Stand der Technik beschreiben Dosisbegrenzungselemente, welche die Injektionsvorrichtung vergrössem, da sie Platz verbrauchen. Insbesondere bei stiftförmigen Injekttonsvorrichtungen nimmt dadurch der Durchmesser der Vorrichtung zu. Mit aus dem Stand der Technik bekannten Injektionsvorrichtungen können Dosen mit einem Dosiseinstellmechanismus eingestellt und anschließend aus einem Produktbehältnis ausgeschüttet werden. Es kann der Fall auftreten, dass mit dem Dosiseinstellelement eine größere Dosis eingestellt wurde, als aus dem Produktbehältnis ausschüttbar ist, z.B. weil das Produktbehältnis eine geringere Dosis enthält als die Dosis, die eingestellt wurde. Dies kann dazu fuhren, dass weniger Produkt ausgeschüttel wird, als eingestellt wurde, was je nach Abweichung zu mehr oder weniger großen Problemen für den Patienten führen kann. Es ist somit eine Aufgabe der Erfindung, einen kompakt gebauten Mechanismus zum Verhindern der Einstellung einer Dosis bereitzustellen, die die Menge eines in dem Produktbehältnis der Injektionsvorrichtung enthaltenen Produkts übersteigt.

Die Aufgabe wird gelöst mit einer Injektionsvorrichtung mit den Merkmalen des Anspruchs 1. Vorteilhafte Weiterentwicklungen ergeben sich aus den abhängigen Ansprüchen und der Beschreibung.

Die Erfindung geht aus von einer Injektionsvorrichtung umfassend ein Dosiseinstellelement, ein damit gekoppeltes erstes Element und einer Kupplung, die bei einer Dosiseinstellung in einem ersten Kupplungszustand ist und bei einer Dosisausschüttung in einem zweiten Kupplungszustand ist, wobei die Kupplung so mit dem ersten Element und mit einem anderen, zweiten Element verbunden ist, dass im ersten Kupplungszustand das erste Element relativ zum zweiten Element drehen kann und im zweiten Kupplungszustand das erste Element relativ zum zweiten Element drehfest ist wobei das erste Element und das zweite Element über ein Koppelglied, das auch als Stoppglied bezeichnet werden kann, gekoppelt sind. Die Injektionsvorrichtung umfasst weiter einen Stoppanschlag, wobei bei einer Dosiseinstellung das Koppelglied eine Bewegung zu einer Stoppposition hin ausführt, wobei das Koppelglied in der Stoppposition im Anschlag mit dem Stoppanschlag die Einstellung einer Dosis verhindert. Weiter weist wenigstens eines aus erstem Element und zweitem Element eine gewindeförmige Führungsbahn auf und weist das andere aus erstem Element und zweitem Element eine gewindeförmige oder eine sich parallel zur Längsachse der Injektionsvorrichtung erstreckende Führungsbahn auf, wobei die Führungsbahn des ersten Elements eine erste Teileinfassung und die Führungsbahn des zweiten Elements eine zweite Teileinfassung bildet, die zusammen eine Einfassung für das Koppelglied bilden, die das Koppelglied zumindest so einfasst, dass ein Schwerpunkt des Koppelglieds dort verbleibt, wo sich die Führungsbahnen überkreuzen.

Das Dosiseinstellelement ist bevorzugt drehbar und kann relativ zum Gehäuse axial fest oder längs bewegbar sein. Zum Beispiel kann das Dosiseinstellelement bei der Drehbewegung eine Längsbewegung, wie z.B. eine Schraubbewegung ausführen. Beispielsweise kann das Dosiseinstellelement von dem Verwender der Vorrichtung für eine Dosiseinstellung relativ zum Gehäuse in zumindest eine Richtung, vorzugsweise für ein Aufdosieren in eine erste und zum Abdosieren, das insbesondere als Dosiskorrektur bezeichnet werden kann, in eine zweite, der ersten Drehrichtung entgegen gesetzte Drehrichtung gedreht werden. Das Dosiseinstellelement kann hülsenförmig sein und sich z.B. im Bereich des proximalen Endes der Injektions Vorrichtung befinden. Alternativ oder zusätzlich kann das Dosiseinstellelement innerhalb des Gehäuses der Vorrichtung angeordnet sein, wobei Gehäuse und Dosiseinstellelement hülsenförmig sein können. Denkbar ist, dass der Verwender nicht unmittelbar Zugriff auf das Dosiseinstelielement hat, sondern über zusätzliche Teile. Grundsätzlich kann das Dosiseinstellelement ein mehrteilig aus z.B. sich rotatorisch oder translatorisch zueinander bewegenden Teilen gebildet sein, wobei auch ein einteiliges Dosiseinstellelement denkbar ist. Das sich innerhalb des Gehäuses befindliche Dosiseinstellelement kann z.B. eine Dosisanzeigehülse sein, die z.B. durch eine Öffnung oder ein Fenster von außen ablesbar ist.

Das erste drehbare Element und/oder das zweite drehbare Element sind bevorzugt hülsenförmig oder/und können um eine gemeinsame Achse drehen, wobei diese Achse beispielsweise in Längsrichtung der Injektionsvorrichtung oder in Richtung der Nadel weist. Erstes Element und zweites Element können konzentrisch zueinander angeordnet sein, wobei bevorzugt das erste Element das zweite Element umgibt oder einen Durchgang für das zweite

Element bildet. Zwischen dem ersten Element und dem zweiten Element kann ein Ringspalt angeordnet sein, in dem z.B. das Koppelglied angeordnet sein kann.

Das Dosiseinstellelement kann mit dem ersten Element mittelbar oder unmittelbar gekoppelt sein. Bevorzugt ist das Dosiseinstellelement mit dem ersten Element beim Einstellen der Dosis drehfest gekoppelt. Diese Kopplung kann beispielsweise bei der Ausschüttung der eingestellten Produktdosis gelöst sein, insbesondere über einen Kupplung. Alternativ kann das Dosiseinstellelement permanent mit dem ersten Element drehfest gekoppelt sein, insbesondere wenn das Dosiseinstellelement eine Dosisanzeigehülse ist, wobei dann die Dosisanzeigehülse relativ zum ersten Element axial verschiebbar sein kann.

Dadurch dass das erste Element bei einer Dosiseinstellung relativ zu dem zweiten Element drehbar ist, kann das Koppelglied relativ zum ersten Element und/oder zum zweiten Element eine Relativbewegung ausführen. Dadurch dass das erste Element bei der Dosisausschüttung relativ zum zweiten Element drehfest ist, kann eine Bewegung des Koppelglieds relativ zu dem ersten Element und dem zweiten Element verhindert werden. Beispielsweise können das erste Element, das zweite Element und das Koppelglied bei der Dosisausschüttung sich gemeinsam insbesondere relativ zum Gehäuse drehen. Bevorzugt ist eine Kupplung vorgesehen, die bei der Dosiseinstellung in einem ersten Kupplungszustand ist und bei der Dosisausschüttung in einem zweiten Kupplungszustand ist. Die Kupplung kann mit dem ersten und mit dem zweiten Element so verbunden sein, dass im ersten, z.B. im geöffneten Kupplungszustand das erste Element relativ zum zweiten Element drehen kann, wobei das erste Element im zweiten Kupplungszustand, insbesondere im geschlossenen Kupplungszustand, relativ zum zweiten Element drehfest ist. Je nachdem, ob der Verwender eine Dosis einstellen oder ausschütten möchte, kann er die Kupplung betätigen, um den gewünschten Kupplungszustand herzustellen.

Das Koppelglied kann bevorzugt formschlüssig in das erste Element und das zweite Element eingreifen. Das erste Element und das zweite Element können jeweils eine Führungsbahn aufweisen, in die das Koppelglied eingreift. Die Führungsbahnen können gewindeförmig oder wendelförmig oder als Längsführung ausgestaltet sein, die in etwa parallel zur Längsrichtung der Injektionsvorrichtung oder zur Drehachse des ersten oder zweiten Elements verläuft.

Es ist zumindest eine der Führungsbahnen gewinde- oder wendelförmig. Die Steigungen für die Gewinde des ersten Elements und des zweiten Elements können unterschiedlich groß und/oder entgegen gesetzt sein, wobei bei letzterem die Gewinde oder Wendeln einen entgegen gesetzten Drehsinn aufweisen. Bevorzugt liegen die Führungsbahnen des ersten Elements und des zweiten Elements gegenüber. Die Führungsbahn des ersten Elements kann z.B. an dem inneren Umfang des ersten Elements, z.B. als Gewinde oder Innengewinde gebildet sein und die Führungsbahn des zweiten Elements kann z.B. an dem äußeren Umfang des zweiten Elements als Gewinde oder Außengewinde gebildet sein. Insbesondere sollen die in Führungsbahnen des ersten Elements und des zweiten Elements nicht ineinander greifen, d.h, kämmungsfrei sein. Lediglich das Koppelglied, wobei auch mehrere Koppelglieder vorgesehen sein können, koppelt die Führungsbahn des ersten Elements mit der Führungsbabn des zweiten Elements.

In bevorzugten Ausführungen kann das Koppelglied eine Kugel sein. Alternativ kann das koppelglied ein Ring, eine Mutter, insbesondere Stoppmutter, oder ein Segment sein. Eine Kugel hat insbesondere den Vorteil, dass sie kein speziell für den Eingriff in die Führungsbahnen ausgestattetes Eingriffsglied aufweisen braucht, sondern durch ihre rotationssymmetrische Form in sämtlichen ihrer möglichen Lagen das erste und das zweite Element koppelt. Die Kugel braucht also nicht speziell ausgerichtet zu werden. Die Führungsbabnen können mit ihrer Querschnittsform an die Kontur der Kugel angepasst sein. Insbesondere kann wenigstens eine der Führungsbahnen, bevorzugt beide, im Querschnitt konkav und/oder abgerundet und/oder in etwa kreisförmig sein.

Ein Ring oder eine Mutter mit einem ringförmigen Querschnitt, bildet einen Durchgang für das zweite Element, und stützt das zweite Element in alle Richtungen quer zu Längsachse zum ersten Element hin ab. An der Mutter sind für jede der Führungsbahmen an ihrem äußeren und inneren Umfang korrespondierende Eingriffselemente, wie z.B. Gewindeabschnitte, für den Eingriff in die jeweilige Führungsbahn gebildet.

Ein segmentförmiges Koppelglied, das insbesondere ein Segment der Stoppmutter sein kann, hat den Vorteil, dass der Ringraum nicht komplett ausgefüllt wird, wodurch Material einerseits und Bauraum andererseits gespart wird. Unter gewissen Bedingungen kann ein Segment besonders montagefreundlich sein.

Das Koppelglied kann in weiteren Beispielen mehrteilig gebildet sein, wie z.B. durch zwei miteinander axial fest und relativ zueinander drehbar verbundene Teile gebildet sein, wovon eines so ausgestaltet ist, dass es in die Führungsbahnen eingreift und das andere beispielsweise einen Anschlag aufweist, der z.B. mit einem später beschriebenen Stoppanschlag in einen Eingriff kommt, um eine weitere Dosiseinstellung zu verhindern. Die das Koppelglied bildenden Teile können beispielsweise um einen bestimmten Winkel, der bevorzugt kleiner ist als 360° und noch bevorzugter kleiner als 180° ist, relativ zueinander verdrehen werden. Die beiden ineinander greifenden Teile können, bei ihrer Bewegung zueinander mittels eines Rastelements mindestens einen, bevorzugt eine Vielzahl, hörbaren oder fühlbaren Klick erzeugen, der dem Verwender der Vorrichtung das Erreichen der Stoppposition anzeigt. Der Klick kann beim Erreichen der Stoppposition oder vor dem Erreichen der Stoppposition erzeugt werden und insbesondere anders klingen oder sich anders anfühlen als andere von der Vorrichtung erzeugte Klick. Bevorzugt wird der Klick beim Weiterdrehen des Dosiseinstellelements um den Winkel, um den die beiden Teile des Koppelglieds relativ zueinander verdrehbar sind, erzeugt. Das Erreichen dieser Stoppposition wird somit vorteilhaft angekündigt. Insbesondere können die beiden Teile des Koppelglieds mit einer Feder gegeneinander vorgespannt sein. Dadurch kann sichergestellt werden, dass die beiden Teile um den bestimmten Winkel zu einander verdrehbar sind, wobei sich dieser Winkel verringert und sich die beiden Teile gegen die Federkraft relativ zueinander verdrehen, wenn sich das Koppelglied in einem Anschlag mit einem Stoppanschlag befindet.

Die Injektionsvorrichtung umfasst ferner einen Stoppanschlag, wobei bei einer Dosiseinstellung das Koppelglied eine Bewegung zu der Stoppposition, insbesondere zu dem Stoppanschlag hin ausführt, wobei das Koppelglied in der Stoppposition, insbesondere im Anschlag mit dem Stoppanschlag die Einstellung einer Dosis verhindert. Insbesondere kann die Erhöhung der Dosis verhindert werden, wobei vorteilhaft die Dosiskorrektur, d.h. Dosisverringerung möglich ist, beispielsweise weil das Koppelglied von dem Stoppanschlag weg bewegt wird. Insbesondere kann das Koppelglied bei der Dosisausschüttung keine Bewegung zu der Stoppposition hin oder von der Stoppposition weg ausführen. Der Stoppanschlag kann von wenigstens einem aus erstem Element, insbesondere einen dreh- oder axialfest mit dem ersten Element verbundenen Element, und zweitem Element, insbesondere einem mit dem zweiten Element axial oder drehfest verbundenen Element gebildet sein. Zum Beispiel kann das Ende der Führungsbahn oder der Führungsbahnen jeweils einen Stoppanschlag bilden.

Grundsätzlich kann der Stoppanschlag in Axialrichtung wirken, wobei bevorzugt ist, dass der Stoppanschlag in Drehrichtung wirkt. Ein in Drehrichtung wirkender Anschlag wirkt der Drehrichtung unmittelbar entgegen. Bei einem in Axialrichtung wirkenden Anschlag wird die Drehbewegung in eine Anpresskraft an den Anschlag übersetzt. Die Anpresskraft des Stoppanschlags an einen in Axialrichtung wirkenden Anschlag ist somit größer als an einen in Drehrichtung wirkenden Anschlag.

Mit der erfindungsgemäßen Anordnung sind beispielsweise mehrere Dosiseinstellungen und Ausschüttungen möglich, wobei bei jeder Dosiseinstellung das Koppelglied ein Stück näher an den Stoppanschlag bewegt wird. Der Abstand, den das Koppelglied zu der Stoppposition aufweist, korrespondiert mit dem Inhalt, den das Produktbehältnis enthält.

Ein Beispiel soll die Anwendung verdeutlichen: Ein Produktbehältnis kann vollständig gefüllt 300 Einheiten Insulin enthalten. Mit jeder einzelnen Dosiseinstellung sind grundsätzlich Werte von 1 bis 60 oder 80 jeweils in einser oder zweier Schritten einstellbar. Bei einem vollständig gefüllten Produktbehältnis ist das Koppelglied einen bestimmten Weg von der Stoppposition entfernt, wobei sich dieser Weg in die Anzahl von in dem Produktbehältnis enthaltenen Einheiten wie in diesem Beispiel 300 Einheiten unterteilen lässt. Mit jeder Dosiseinstellung wird das Koppelglied der eingestellten Dosis entsprechend weit in Richtung Stoppposition bewegt. Sind beispielsweise 295 Einheiten aus dem Produktbehältnis ausgeschüttet, verbleiben noch 5 Einheiten in dem Produktbehältnis. Dementsprechend ist auch das Koppelglied 5 Einheiten entfernt von der Stoppposition. Obwohl mit dem Dosiseinstellelement grundsätzlich für jede Dosiseinstellung 60 oder 80 Einheiten einstellbar sind, kann in diesem Fall das Dosiseinstellelement bloß um 5 Einheiten aufdosiert werden, da dann das Koppelglied in den Anschlag mit dem Stoppanschlag gerät, wodurch eine Dosiserhöhung verhindert wird. Dadurch kann die Gefahr einer Fehlanwendung der Injektionsvorrichtung verringert werden.

In bevorzugten Ausführungen kann das Koppelglied so mit dem ersten Element und dem zweiten Element gekoppelt sein, dass das Koppelglied bei einer Bewegung des ersten Elements relativ zum zweiten Element relativ zu wenigstens einem, vorzugsweise beiden aus dem ersten Element und dem zweiten Element drehbar ist oder gedreht wird. Beispielsweise kann bei einer Drehung des ersten Elements relativ zum zweiten Element das Koppelelement drehfest mit entweder dem ersten oder dem zweiten Element sein und sich zusammen mit dem ersten oder zweiten Element relativ zu dem anderen aus erstem und zweitem Element drehen. Hierzu kann das Koppelglied in eine Längsführung eingreifen, die bevorzugt an dem Element gebildet ist, relativ zu dem das Koppelglied bei der Dosiseinstellung drehfest ist. Das Koppelglied kann außerdem in eine gewindeförmige Führungsbahn, insbesondere ein Gewinde, eingreifen, die an dem Element gebildet ist, relativ zu dem sich das Koppelglied zusammen mit dem anderen Element bei der Dosiseinstellung dreht.

In der Ausführung in der sich das Koppelglied bei der Drehbewegung des ersten Elements relativ zum zweiten Element relativ zum ersten und zum zweiten Element dreht, kann das Koppelglied in das Gewinde des ersten Elements und in das Gewinde des zweiten Elements eingreifen. Hierdurch wird bei einer Drehung das Koppelglied kontinuierlich oder übersetzt oder untersetzt gedreht, insbesondere um die Längsachse, um die sich das erste und zweite Element drehen können. Der Vorteil hierbei ist, dass, egal ob das Dosiseinstellelement oder das erste Element relativ zu dem zweiten Element eine viertel, eine halbe, eine ganze oder mehrere Umdrehungen ausführt, das Koppelglied stets relativ zum ersten und zum zweiten Element eine Drehbewegung ausführt. Die Erfindung unterscheidet sich hierbei von Zählringen, bei denen beispielsweise ein Einerzählring um eine volle Umdrehung relativ zu einem Zehnerzählring gedreht wird und am Ende der vollen Umdrehung den Zehnerzählring um eine Einheit mitnimmt. Das Teil des Einerzählrings, das dem Zähnerzählring mitnimmt, führt zum Zähnerzählring keine Relativbewegung aus, sondern zum Zähnerzählring und dies nur, wenn der Einerzählring den Zähnerzählring nicht mitnimmt.

Vorteilhaft an der Erfindung ist ferner, dass das Koppelglied bei einer Drehung des ersten Elements relativ zu dem zweiten Element um einen Drehwinkel mitgedreht wird, der größer oder vorzugsweise kleiner als der Drehwinkel des ersten Elements ist. Dies gilt auch für die Drehwinkelgeschwindigkeit, d.h. dass bei einer Drehung des ersten Elements relativ zu dem zweiten Element das Koppelglied relativ zu dem Gehäuse um eine Drehwinkelgeschwindigkeit mitgedreht wird, die größer oder vorzugsweise kleiner ist als die Drehwinkelgeschwindigkeit des ersten Elements. Bevorzugt ist der Drehwinkel oder die Drehwinkelgeschwindigkeit des Koppelglieds größer als der oder die des zweiten Elements, da dieses zum Gehäuse bei der Dosiseinstellung besonders bevorzugt stillsteht.

Beispielsweise ist der Drehwinkel oder die Drehgeschwindigkeit des Koppelglieds, das oder die es bei einer Drehung des ersten Elements relativ zu dem zweiten Element oder dem Gehäuse ausführt, größer, wenn die Gewinde oder Wendeln den gleichen Drehsinn aufweisen und die Steigung der Führungsbahn des ersten Elements größer ist als die Steigung der Führungsbahn des zweiten Elements. Das Koppelglied führt relativ zu dem zweiten Element oder dem Gehäuse einen geringeren Drehwinkel oder eine langsamere Drehwinkelgeschwindigkeit aus als das erste Element relativ zum zweiten Element oder dem Gehäuse, wobei die Drehrichtung des Koppelglieds entgegengesetzt zur Drehrichtung des ersten Elements relativ zum zweiten Element ist, wenn die Gewinde oder Wendeln den gleichen Drehsinn aufweisen und die Führungsbahn des ersten Elements eine kleinere Steigung aufweist als die Führungsbahn des zweiten Elements. Die Führungsbahnen des ersten Elements und des zweiten Elements können grundsätzlich den gleichen Drehsinn und den gleichen Steigungen aufweisen.

Das Koppelglied kann relativ zu dem zweiten Element oder dem Gehäuse in die gleiche Drehrichtung und um einen geringeren Drehwinkel oder eine geringere Drehwinkelgeschwindigkeit drehen wie das erste Element relativ zum zweiten Element oder dem Gehäuse, wenn die Gewinde oder wendelförmigen Führungsbahn des ersten Elements und des zweiten Elements einen entgegengesetzten Drehsinn aufweisen. Die Steigungen der Führungsbahnen des ersten Elements und des zweiten Elements können gleichgroß sein oder die Steigung der Führungsbahn des ersten Elements kann größer sein als die des zweiten Elements oder umgekehrt.

Die Steigungen der Gewinde oder wendelförmigen Führungsbahnen können sehr klein oder sehr groß sein, wobei bei einer endlich großen Steigung die Führungsbahn eine sich entlang der Drehachse, d.h. parallel zur Drehachse erstreckende Führungs- bzw. Längsführungsbahn handelt.

Das erste drehbare Element kann mit einer Feder, insbesondere einer Torsionsfeder oder Drehfeder verbunden sein, die die zur Dosisausschüttung notwendige Energie speichert und bei Bedarf abgibt. Bevorzugt kann durch die Dosiseinstellbewegung des Dosiseinstellelements oder des ersten Elements die Feder gespannt werden. Grundsätzlich kann die Feder wendelförmig oder vorzugsweise spiralförmig sein. Die Feder kann aus einem Draht oder vorzugsweise aus einem bandförmigen Material, insbesondere Federstahl gewickelt sein. Solche Federn werden auch als Uhrenfedern bezeichnet. Wenigstens eines aus Dosisanzeigehülse, erstem Element, und Dosiseinstellelement können bei der Dosiseinstellung drehfest mit einem Ende der Feder gekoppelt sein, wobei das andere Ende der Feder vorteilhaft mit dem Gehäuse verbunden ist. Für die Dosisausschüttung können das erste Element und/oder die Dosisanzeigehülse drehfest mit einem Ende der Feder gekoppelt sein, wobei das Dosiseinstellelement beispielsweise entkoppelt ist.

Die Dosisanzeigehülse kann z.B. ein Gewinde, insbesondere ein Innen- oder Außengewinde aufweisen, das in das Gehäuse oder in ein gehäusefestes Element eingreift, um für die Dosisanzeige eine Schraubbewegung ausführen zu können. Hierfür kann die Dosisanzeigehülse eine an ihrem äußeren Umfang angeordnete wendelförmige Skala aufweisen. Die Dosisanzeigehülse kann Anschläge, insbesondere in Axialrichtung oder vorzugsweise in Umfangsrichtung wirkende Anschläge aufweisen, die in entsprechende Gegenanschläge mit z.B. dem Gehäuse anschlagen können. Die Dosisanzeigehülse kann sich zwischen diesen Anschlägen hin und her bewegen, so dass mit ihr Dosen von Null bis zur gewünschten Maximaldosis, wie z.B. 60 oder 80 Einheiten einstellbar sind, insbesondere sofern sich das Koppelglied nicht in der Stoppposition befindet. Somit kann eine maximal einstellbare Dosis einerseits mit dem Anschlag der Dosisanzeigehülse oder mit dem Koppelglied begrenzt werden, je nachdem welches der beiden Elemente zuerst eine Dosiserhöhung verhindert.

Wie allgemein bevorzugt kann das zweite drehbare Element drehfest mit einem Abtriebsglied, insbesondere einer Kolbenstange gekoppelt sein, wobei das Abtriebsglied insbesondere für eine Produktausschüttung in Ausschüttrichtung schraubbar ist. Hierzu kann das Abtriebsglied beispielsweise ein Gewinde aufweisen, mit dem es in das Gehäuse oder ein gehäusefestes Element eingreift, um die Schraubbewegung ausführen zu können. Beispielsweise kann das zweite drehbare Element auch die Kolbenstange sein. Besonders bevorzugt ist das zweite drehbare Element hülsenförmig und drehfest mit dem Abtriebsglied gekoppelt, wobei das Abtriebsglied eine Längsbewegung relativ zu dem zweiten drehbaren Element ausführen kann. Das zweite drehbare Element kann mittelbar oder unmittelbar mit dem Abtriebsglied gekoppelt sein. Beispielsweise kann das zweite Element über eine Hülse, insbesondere eine Kupplungshülse drehfest oder längs verschiebbar mit dem Abtriebsglied gekoppelt sein. Das zweite Element kann drehfest und längs verschiebbar mit der Hülse, die zwischen Abtriebsglied und zweiten Element angeordnet ist, gekoppelt sein, wie z.B. über Längsnuten. Dies hat den Vorteil, dass bei einem Produktbehältniswechsel das zweite Element relativ zum ersten Element keine Axialbewegung ausführen braucht oder ausführt, wobei das AbtriebsgHed und gegebenenfalls die das Abtriebsglied umgebende Hülse axial relativ zu dem zweiten Element bewegbar sein können. Insbesondere kann die unmittelbar in das Abtriebsglied eingreifende Hülse das zweite Element sein.

Erfindungsgemäss weist wenigstens eines aus erstem Element und zweitem Element eine gewindeförmige Führungsbahn oder Nut auf, und das andere aus erstem Element und zweitem Element weist eine gewindeförmige oder sich parallel zur Längsachse erstreckende Führungsbahn oder Nut auf, wobei das Koppelglied, insbesondere dessen Schwerpunkt stets dort angeordnet ist, wo sich die Führungsbahnen oder Nuten insbesondere in ihrer Projektion über kreuzen. Vorteilhaft kann erreicht werden, dass der Ringspalt zwischen erstem Element und zweiten Element relativ dünn ist. Die Führungsbahn oder Nuten bilden eine Einfassung für das Koppelglied die das Koppelglied zumindest so einfasst, dass es dort verbleibt, wo sich die Führungsbahnen überkreuzen. Das Koppelglied kann mit einem mehr oder weniger großen Spiel in dieser Einfassung frei bewegbar sein. In der Projektion der beiden Führungsbahnen kann die Einfassung kann z.B. parallelogrammförmig, insbesondere rechteckig, quadratisch oder
rautenförmig sein. Die sich gegenüberliegenden Kanten des Parallelogramms werden von jeweils einer der Führungsbahnen gebildet Die Einfassung stellt sicher, dass das Koppelglied dort verbleibt, wo sich die Führungsbahnen überkreuzen. Bei einer Verdrehung des ersten Elements relativ zum zweiten Element wandert die Einfassung und somit das sich darin befindliche Koppelglied relativ zum ersten oder/und zum zweiten Element. Insbesondere wird das Koppelglied bei einer Dosiserhöhung mittels der Einfassung zur Stoppposition hin geführt und bei einer Dosisverringerung von der Stoppposition weg geführt. Die Führungsbahn des ersten Elements bildet eine erste Teileinfassung und die Führungsbahn des zweiten Elements bildet eine zweite Teileinfassung, die zusammen die Einfassung für das Koppelglied bilden. Das Koppelglied kann im Wesentlichen vollständig innerhalb der Einfassung angeordnet sein, zumindest ist der geometrische Schwerpunkt oder der Massenschwerpunkt des Koppelglieds innerhalb der Einfassung angeordnet. Als besonders vorteilhaft hat sich hier ein kugelförmiges Koppelglied herausgestellt, Alternativ könnten auch andere rotationssymmetrische Körper, wie z.B. ein zylindrischer, in Frage kommen, insbesondere wenn eine der beiden Führungsbahnen eine Längsführung ist. Denkbar sind auch andere Körper, die in die Einfassung passen, wie z.B. Würfel oder Körper mit einem parallelogrammförmigen insbesondere rautenförmigen, rechteckigen oder quadratischen Querschnitt Allgemein bevorzugt ist der Stoppanschlag an den das Koppelglied in der Stoppposition anschlägt an die Anschlagfläche des Koppelglieds angepasst, um möglichst eine Flächenpressung zu erzeugen. Zum Beispiel kann der Stoppanschlag für ein kugelförmiges Koppelglied ein Teil einer Kugelschale sein, die bevorzugt den gleichen Radius aufweist wie das kugelförmige Koppelglied.

Insbesondere bildet wenigstens eine der Führungsbahnen oder Nuten einen in Axialrichtung oder Umfangsrichtung wirkenden Anschlag, d.h. den Stoppanschlag für das Koppelglied. Zum Beispiel kann das Ende der Führungsbahn des ersten Elements in der Stoppposition an das Koppelglied anschlagen, wobei das Koppelglied an das Ende der Führungsbahn des zweiten Elements anschlägt. Das Koppelglied kann zwischen den Enden der Führungsbahnen des ersten und zweiten Elements eingeklemmt werden. Eine Verdrehung des ersten Elements relativ zum zweiten Element für eine Dosiserhöhung wird verhindert. Grundsätzlich könnte auch eine Flanke einer der Längsführungen einen Anschlag bilden, wobei dies besonders bevorzugt ist bei einer als Längsführung ausgebildeten Führungsbahn.

In weiteren Beispielen kann das Koppelglied sich nur teilweise über den Umfang des zweiten drehbaren Elements erstrecken. Ein solches Koppelglied kann grundsätzlich eines der oben genannten für die Einfassung vorgesehenen Körper sein, wobei bevorzugt ist, dass das Koppelglied sich segmentförmig über den Umfang des zweiten Bahnelements erstreckt. Das Segment kann z.B. ein Teil des Umfangs einer Mutter sein. Die beiden in Umfangsrichtung weisenden Enden, d.h. die entgegengesetzten Enden des segmentförmigen Koppelglieds können das zweite drehbare Element bevorzugt so weit umgreifen, dass die Verbindungsgerade durch die beiden Enden den Umfang des von dem zweiten Element gelagerten Abtriebsglieds, wie z.B. der Kolbenstange, passiert oder tangiert. Dadurch, dass das Segment keinen Durchgang für das Abtriebsglied bildet, kann Platz in dem Ringspalt zwischen dem ersten und dem zweiten Element gespart werden. Der Vorteil hierbei ist, dass in dem Ringspalt andere Teile der Injektionsvorrichtung angeordnet werden können, wodurch die Injektionsvorrichtung insgesamt kompakter gebaut werden kann. Das Segment kann wie eine Mutter auch, an seiner Stirnfläche einen Anschlag für den Stoppanschlag aufweisen, der besonders bevorzugt in Drehrichtung weist oder wirkt.

Zum Beispiel kann in dem Ringspalt bezogen auf den Umfang des zweiten Elements dem Koppelglied gegenüberliegend. ein Stützglied angeordnet sein. Das Stützglied kann als separates Teil in den Ringspalt eingelegt sein oder an einem aus erstem Element, zweitem Element und Koppelglied gebildet sein. Beispielsweise kann das Stützglied an dem zweiten Element in den Ringspalt ragend gebildet sein, wobei das Koppelglied an dem zweiten Element längsgeführt ist. Das Stützglied kann sich hierbei an dem inneren Umfang des ersten Elements abstützen bzw. dort entlang gleiten. Das Stützglied kann alternativ an dem ersten Element gebildet sein und in den Ringspalt ragen, wobei sich das Koppelglied an dem ersten Element längs geführt ist. Das Stützglied kann sich hierbei an dem äußeren Umfang des zweiten Elements abstützen, insbesondere daran entlang gleiten. Durch ein Stützglied kann vorteilhaft erreicht werden, dass bei einer unsachgemäßen Verwendung, z.B. wenn der Verwender mit Gewalt an dem Dosiseinstellelement dreht das erste oder das zweite Element nicht aus ihren konzentrischen Anordnungen ausgelenkt werden können, wenn beispielsweise das Koppelglied stark an den Stoppanschlag gepresst wird.

Alternativ oder zusätzlich zu dem Stützglied kann der Anschlag des Koppelglieds oder der Stoppanschlag oder können beide eine entsprechende Form aufweisen, die verhindert, dass das erste oder/und zweite Element aus seiner Drehachse ausgelenkt wird.

In weiteren Beispielen kann das Koppelglied kraft-, form- oder/und stoffschlüssig, insbesondere mit mindestens einer Sollbruchstelle mit einem Basiselement verbunden sein, wobei diese Verbindung bei der erstmaligen Verdrehung des ersten Elements relativ zu dem zweiten Element lösbar ist. Beispielsweise kann das Basiselement in den Ringspalt zwischen dem ersten und zweiten Element angeordnet sein. Möglich ist auch eine Ausführungsform, in der das Basiselement das Stützglied bildet. Basiselement und Koppelglied können in einer Ausgangsposition, insbesondere vor einer ersten Verwendung der Injektionsvorrichtung einteilig gebildet sein, insbesondere durch ein Spritzgussverfahren. Es können Stege zur Bildung von Sollbruchstellen vorgesehen sein, die bei einer Bewegung des ersten Elements relativ zum zweiten Element brechen, indem z.B. das Koppelglied relativ zu dem Basiselement axial oder/und drehbewegt wird. Das Basiselement dient vorteilhaft als Montagehilfe für das Koppelglied, insbesondere das segmentförmige Koppelglied.

In weiteren Beispielen kann das Koppelglied sich zumindest teilweise, insbesondere vollständig mit dem Umfang des zweiten drehbaren Elements erstrecken, wie z.B. eine Mutter bilden. Bevorzugt sind in dieser Ausführungsform zwei gewindeförmige Führungsbahnen vorgesehen. Für diese Ausführungsform ist es bevorzugt, dass die Steigung eines der beiden gewindeförmigen Führungsbahnen in die das Koppelglied eingreift größer ist als die Steigung des anderen der beiden gewindeförmigen Führungsbahnen. Entsprechend der hierin genannten Beispiele der sich überkreuzenden Führungsbahnen für das Koppelglied ein gleicher oder gegenläufiger Drehsinn oder eine Über- oder Untersetzung erzeugt werden, wobei die Bewegung des Koppelglieds kontinuierlich ist.

Insbesondere kann die Drehung des Koppelglieds relativ zum ersten Element oder zum zweiten Element kleiner sein als eine volle Umdrehung, wenn das Koppelglied von der Position, in der es am weitesten von der Stoppposition entfernt ist, in die Stoppposition bewegt wird und hierfür kann wenigstens eine der Führungsbahnen eine sehr große Gewindesteigung aufweisen. Hierdurch wird eine vorteilhafte Reibungssituation in der Injektionsvorrichtung erreicht. Das Koppelglied ist insbesondere bei einem vollen Produktbehältnis am weitesten von der Stoppposition entfernt.

In bestimmten Ausführungsformen kann es von Vorteil sein, wenn die Führungsbahnen eine Breite aufweist, die sich zu der Stoppposition aufweitet oder verjüngt, wodurch ein Spiel für das Koppelglied bei der Bewegung in die Stoppposition erhöht oder verringert werden kann. Außerdem ergeben sich hierbei Vorteile in der Herstellung des zweiten Elements, z.B. bei der Entformung aus einer Spritzgussform.

Die Erfindung wurde anhand mehrerer Ausführungsformen beschrieben. Im Folgenden werden bevorzugte Ausführungen der Erfindung anhand von Figuren beschrieben. Die hierbei offenbarten Merkmale bilden je einzeln und in Kombination den Gegenstand der Erfindung vorteilhaft weiter. Es zeigen:
- Figur 1: eine Querschnittsansicht eines proximalen Teils einer Injektionsvorrichtung,
- Figur 2: eine perspektivische Ansicht eines Abtriebsglieds mit Flansch und Federglied,
- Figur 3: eine perspektivische Ansicht einer modifizierten Kupplungshülse für die Vorrichtung aus Figur 1,
- Figur 4: eine perspektivische Ansicht einer weiteren Modifikation für die Vorrichtung aus Figur 1,
- Figur 5: eine Querschnittsansicht mit der Modifikation aus Figur 4,
- Figuren 6 und 7: perspektivische Ansichten einer weiteren Modifikation für die Vorrichtung aus Figur 1,
- Figur 8: eine Querschnittsansicht mit der Modifikation aus den Figuren 6 und 7,
- Figuren 9 und 10: perspektivische Ansichten einer weiteren Modifikation für die Vorrichtung aus Figur 1,
- Figur 11: einer Querschnittsansicht mit der Modifikation aus den Figuren 9 und 10,
- Figur 12: eine perspektivische Ansicht einer Weiterbildung der Modifikation aus den Figuren 9 bis 11,
- Figur 13: eine perspektivische Ansicht einer weiteren Modifikation für die Vorrichtung aus Figur 1,
- Figur 14: eine perspektivische Ansicht einer weiteren Modifikation für die Vorrichtung aus Figur 1 und
- Figur 15: eine Querschnittsansicht eines mehrteiligen Koppelglieds.

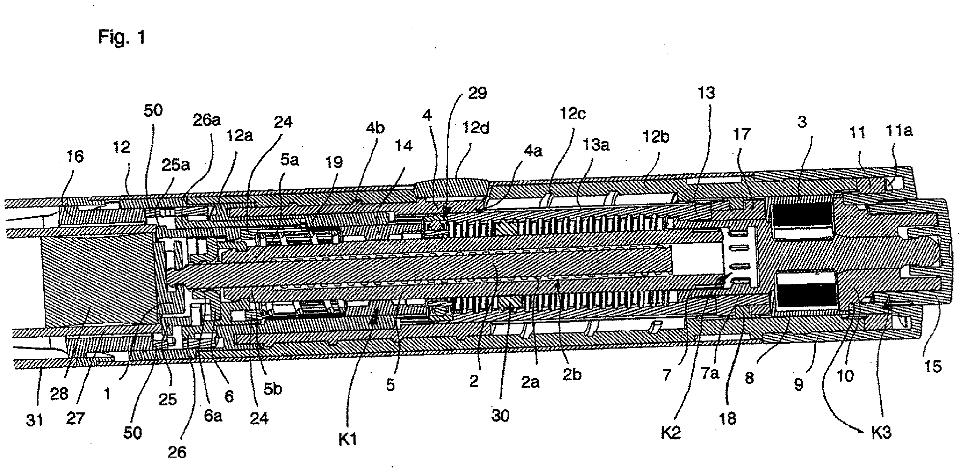

Die in Figur 1 gezeigte Injektionsvorrichtung umfasst eine Antriebseinheit, die bevorzugt mehrfach verwendbar ist, und ein damit verbundenes Produktbehältnis 27, das in einer hülsenförmigen z.B. mehrfach verwendbaren Produktbehältnisaufnahme 16 aufgenommen und mit Hilfe der Produktbehältnisaufnahme 16 an der Antriebseinheit befestigbar ist. Das Produktbehältnis 27 kann nach seiner Entleerung von der Injektionsvorrichtung entfernt, entsorgt und gegen ein neues ausgetauscht werden. Das Gehäuse 12 ist wegen der einfacheren Herstellbarkeit und Montierbarkeit mehrteilig gebildet mit damit verbundenen oder eingesetzten Elementen 12a, 12b, wobei das Gehäuse grundsätzlich auch einteilig gebildet sein könnte. Das Produktbehältnis 16 ist mit Hilfe eines Bajonettverschlusses, der von dem Gehäuse 12, der Produktbehältnisaufnahme 16 und der Hülse 50 gebildet wird, an der Antriebseinheit befestigt. Die Produktbehältnisaufnahme 16 wird durch eine Kappe 31 abgedeckt, die an das Gehäuse 12 angesteckt, für eine Verwendung der Injektionsvorrichtung abnehmbar und anschließend wieder aufsteckbar ist.

Zum Anbringen des Produktbehältnisses 27 an die Antriebseinheit wird ein neues Produktbehältnis 27 durch das proximale Ende in die Produktbehältnisaufnahme 16 eingeführt. Anschließend wird die Produktbehältnisaufnahme 16 mit einer axialen Bewegung oder einer kombinierten Dreh- und Axialbewegung drehmomentfest an die Hülse 50 angesteckt.

In der Hülse 50, die auch als Bajonetthülse bezeichnet werden kann, ist eine Führungshülse 26 aufgenommen. Die Führungshülse 26 ist mit dem Gehäuse 12 drehfest und axial bewegbar sowie mit der der Bajonetthülse 50 drehbar und axial fest verbunden. Dies bewirkt, dass bei der Bewegung der Bajonetthülse 50 aus der entriegelten in die verriegelte Position und umgekehrt, die Führungshülse 26 eine längsgeführte Bewegung relativ zum Gehäuse 12 ausführt.

Wie in Figur 1 zu erkennen ist, ist ein Gewindeeinsatz 6 dreh- und axialfest mit der Führungshülse 26 verbunden, insbesondere verrastet. Gewindeeinsatz 6 und Führungshülse 26 können als Eingriffsglied 6, 26 bezeichnet werden. Der Gewindeinsatz 6 weist ein Innengewinde 6a auf, in welchem das Außengewinde 2a eines Abtriebsglieds 2, das in diesem Beispiel auch als Kolbenstange bezeichnet werden kann, geführt wird, so dass, wenn das Abtriebsglied 2 gedreht wird, sich dieses geführt durch das Innengewinde 6a des Gewindeeinsatzes 6 in Abhängigkeit von der Drehrichtung entweder in proximale Richtung oder in distale, d.h. entgegengesetzte Richtung schraubt.

Das Abtriebsglied 2 trägt auf seiner Außenseite ein Gewinde 2a welches von zwei am Umfang gegenüberliegenden, in axiale Richtung verlaufenden Nuten 2b unterbrochen wird. Eine Kupplungshülse 5, die Teil eines Übertragungselements 7, K2, 5 ist, hat an ihrem distalen Ende zwei einander gegenüberliegende radial nach innen gerichtete Abragungen 5a, 5b, welche in die Nuten 2b des Abtriebsglieds 2 ragen. Die Kupplungshülse 5 ist mit dem Eingriffsglied 6, 26 drehbar und axial fest verbunden. Somit ist das Abtriebsglied 2 relativ zu der Kupplungshülse 5 verdrehgesichert und kann relativ zur Kupplungshülse 5 axial bewegt werden, wenn es relativ zu dem Eingriffsglied 2, 26 gedreht wird. Außer während eines Austauschs des Produktbehältnisses 27 ist die Kupplungshülse 5 axial nicht verschiebbar.

Eine an dem proximalen Ende der Injektionsvorrichtung vorgesehene Antriebswelle 7, die Teil des Übertragungselements 7, K2, 5 ist, weist radial nach innen ragende Zähne 7a auf, welche ein Kupplungselement der Kupplung K2 bilden. Durch das Betätigungen, d.h. Drücken eines Betätigungselements 15 in distale Richtung, werden die Antriebswelle 7 und dadurch auch die Zähne 7a in distale Richtung verschoben, wodurch die Zähne 7a in das proximale Ende der Kupplungshülse 5 eingreifen und eine drehmomentfeste, insbesondere formschlüssige Verbindung bilden.

Ein Federelement bzw. eine Antriebsfeder 3, welche in Form einer Spiralfeder oder Uhren-Feder ausgebildet sein kann, ist mit einem Ende mit dem Gehäuse 12 über einer Federhülse 8 auf der Außenseite des Federelements 3 verbunden. Die Federhülse 8 ist relativ zu dem Gehäuse 12 verdrehgesichert und axial verschiebbar. Am anderen Ende ist die Antriebsfeder 3 mit der Antriebswelle 7 verbunden. Hierdurch kann eine in dem Federelement 3 gespeicherte Energie als Drehbewegung der Antriebswelle 7 relativ zu dem Gehäuse 12 abgegeben werden. Für eine Produktausschüttung wird die Energie des Federelements 3 über das Übertragungselement 5, K2, 7 in Form einer Drehbewegung an das Abtriebsglied abgegeben, so dass sich dieses relativ zu dem Eingriffsglied 6, 26 in distale Richtung, d.h. in Ausschüttrichtung schraubt und den Kolben 28 verschiebt, der das Produkt aus dem Produktbehältnis 27 ausschüttet.

Zum Einstellen einer zu verabreichenden Produktdosis kann ein Benutzer das als Dosierknopf ausgestaltete Dosierelement 9, das relativ zu dem Gehäuse 12 axialfest ist, drehen. Das Dosierelement 9 ist über die Kupplung K3 mit einem Kupplungsglied 10 verdrehgesichert gekoppelt. Die Kupplung K3 wird gebildet durch Stege oder Nuten oder Zähne des Dosierknopfes 9, welche mit Stegen oder Nuten oder Zähnen der Kupplungsscheibe 10 formschlüssig zusammenwirken, um eine durch Verschieben des Kupplungsglieds 10 in distale Richtung lösbare Kupplung zu bilden. Das Kupplungsglied 10 kann durch Betätigen des Betätigungselements 15 verschoben und somit gelöst werden. In einem unbetätigten Zustand werden die Kupplungen K3 in einem eingekuppelten und die Kupplung K2 in einem ausgekuppelten Zustand gehalten mittels eines Federelements 19, welches die Antriebswelle 7 in proximale Richtung drückt. Während des Dosiseinstellvorgangs ist die Kupplung K3 eingekuppelt, d.h. eine Drehbewegung des Dosierknopfes 9 wird auf das Kupplungsglied 10 übertragen. Das Kupplungsglied 10 ist mit der Antriebswelle 7 axial- und drehfest verbunden und könnte auch einteilig mit der Antriebswelle 7 ausgebildet sein. Die Drehbewegung des Dosierglieds 9 wird aufgrund der ausgekuppelten Kupplung K2 nicht auf die Kupplungshülse 5 übertragen.

Durch Drehung der Antriebswelle 7 wird die mit der Antriebswelle 7 verbundene Antriebsfeder 3 gespannt. Um zu verhindern, dass durch die während des Einstellvorganges gespannte Antriebsfeder 3 der Dosierknopf 9 wieder zurückgedreht wird, ist eine Ratsche 11 oder ein Ratschmechanismus, welcher eine Ratschenfeder 11a z.B. zum Spannen von Halteelementen aufweisen kann, zwischen dem Gehäuse 12 der Injektionsvorrichtung, dessen Bestandteile z.B. ein Mechanikhalter 12a und ein Mechanikhalter 12b sein können, und dem Dosierknopf 9 vorgesehen. Der Ratschenmechanismus kann so ausgestaltet sein, dass nur die Drehung in eine Richtung, insbesondere nur ein Spannen der Antriebsfeder 3 möglich ist. Bevorzugt wird jedoch, dass der Ratschenmechanismus so ausgestaltet ist, dass die Drehung in beide Drehrichtung, insbesondere ein Spannen und Entspannen der Antriebsfeder 3 möglich ist. Durch eine Drehbarkeit in beide Richtungen kann bei der Einstellung der Produktdosis sowohl eine Produktdosis erhöht als auch verringert werden. Die aktuell eingestellte Produktdosis kann über das Fenster 12d von einer Anzeigetrommel 4 abgelesen werden.

Die Drehbewegung der Antriebswelle 7 wird auch auf die Gewindehülse 13 übertragen, welche dreh- und axialfest mit der Antriebswelle 7 verbunden ist und auch einteilig mit dieser ausgebildet sein könnte. Die Gewindehülse 13 trägt auf ihrem äußeren Umfang 13a mindestens eine Nut, in welche mindestens ein Steg 4a der Anzeigetrommel 4 eingreift, so dass eine Drehbewegung der Gewindehülse 13 auf die Anzeigetrommel 4 durch die verdrehsichere Kopplung übertragen wird, wobei eine axiale Relativbewegung zwischen Anzeigetrommel 4 und Gewindehülse 13 möglich ist. Die Anzeigetrommel 4 weist auf ihrer Außenseite ein Gewinde 4b auf, welches in ein Innengewinde 12c des Gehäuseteils 12b eingreift, so dass die Anzeigtrommel 4 durch eine Drehbewegung in axiale Richtung relativ zum Gehäuse 12 vorzugsweise in distale Richtung verschoben wird. Bevorzugt bewegt sich die Anzeigetrommel 4 während eines Einstell- oder Aufdosiervorganges durch Drehung des Dosierknopfes 9 in distale Richtung der Injektionsvorrichtung (in Fig. 1 nach links). Auf der Außenseite der Anzeigetrommel 4 kann eine Markierung, wie z.B. eine Beschriftung, eine Dosisanzeige oder eine Skala vorgesehen sein, welche durch eine Durchbrechung oder ein Fenster 12d im Gehäuse 12b der Injektionsvorrichtung ablesbar ist, wobei sich die Markierung der Anzeigetrommel 4 relativ zum Fenster 12d verschiebt. Die Anzeigetrommel 4 weist an seinem distalen Ende einen in Umfangsrichtung wirkenden Drehanschlag auf, der bei maximaler Dosis in einen Anschlag mit einem entsprechend an dem Gehäuseteil 12a ausgebildeten Gegenanschlag gerät. Der Gegenanschlag wird von einem stirnseitigen Ende eines Ringspalts des Gehäuseteils 12a gebildet. Ein in Umfangsrichtung wirkender Anschlag hat gegenüber einem Axialanschlag den Vorteil, dass geringere Kräfte auf den Anschlag wirken. Die Anzeigetrommel 4 weist ferner an seinem proximalen Ende einen weiteren in Umfangsrichtung wirkenden Drehanschlag auf, der bei minimaler Dosis in einen Anschlag mit einem entsprechend an dem Gehäuse 12b ausgebildeten Gegenanschlag gerät. Der Gegenanschlag wird von dem proximalen Ende des Gewindegangs 12c gebildet.

Nach dem Einstellen der Dosis und Aufziehen der Antriebsfeder 3 durch Drehen des Dosierknopfes 9 ist der Einstellvorgang beendet, wobei bevorzugt ist, dass das Spannen der Feder 3 beim Aufdosieren erfolgt. Zur Dosiskorrektur kann der Dosierknopf 9 einfach in Gegenrichtung gedreht werden, um eine eventuell zu groß eingestellte Dosis wieder zu verkleinern.

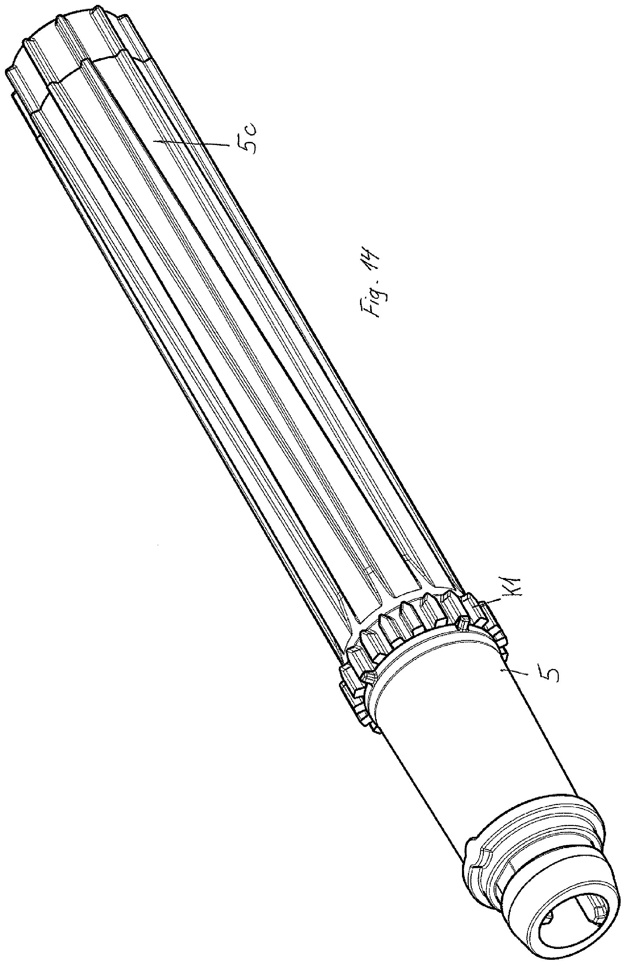

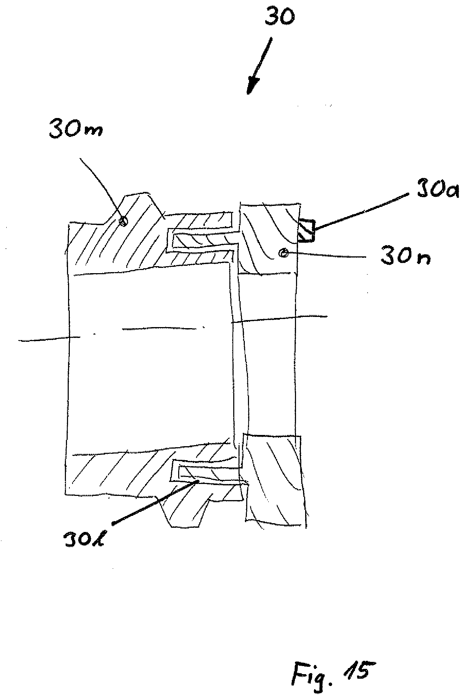

Die Ratsche 11 kann so ausgebildet sein wie in den Figuren 14 und 15 der Patentanmeldung PCT/CH2007/000243 beschrieben, deren diesbezügliche Lehre in die Patentanmeldung aufgenommen wird.

Während eines Ausschüttvorganges, welcher durch Drücken auf den Druckknopf 15 ausgelöst wird, wird die Anzeigetrommel 4 wieder in Gegenrichtung gedreht und verschiebt sich durch den Gewindeeingriff mit dem Innengewinde 12c der Injektionsvorrichtung wieder zurück in proximale Richtung (in Fig. 1 nach rechts). Dabei kann es auch zu einem in Umfangsrichtung wirkenden Anschlag der Anzeigetrommel 4 an das Gehäuse 12a, 12b der Injektionsvorrichtung und insbesondere an dem Gehäuseteil 12b kommen. Dieser Vorgang kann bei einer ungebremsten Ausschüttbewegung, bei welcher die Gewindestange 2 ohne Gegenkraft in distale Richtung bewegt wird, z.B. wenn kein Produktbehältnis eingelegt ist, zu einer starken Belastung und im Extremfall zu einer Verformung oder sogar Beschädigung der Anzeigetrommel 4 oder des Gegenstücks 12b führen. Es ist daher eine auf die Antriebsbewegung wirkende Bremseinrichtung 17, 18 vorgesehen.

Die Kupplung K1, die aus dem als Arretierhülse 14 ausgestalteten Kupplungsglied und der Kupplungshülse 5 gebildet wird, dient dazu, in bestimmten Schaltzuständen die Kupplungshülse 5 drehfest mit dem Gehäuse 12 zu koppeln bzw. für eine Drehung relativ zu dem Gehäuse 12 zu entkoppeln. Die Kupplung K1 ist bevorzugt entkoppelt bei einem Austausch des Produktbehältnisses 27, um das Abtriebsglied 2 wieder in proximale Richtung zurückschieben bzw. schrauben zu können, und bei einer Produktausschüttung, um das Abtriebsglied 2 in distale Richtung schrauben zu können. Die Kupplung K1 ist bevorzugt eingekuppelt, wenn das Produktbehältnis an der Antriebseinheit befestigt ist und das Betätigungselement 15 unbetätigt ist. Die Kupplung K1 wird gebildet durch Zähne auf der Außenseite der Kupplungshülse 5, welche in Zähne auf der Innenseite der Arretierhülse 14 eingreifen. Hierdurch wird die Kupplungshülse 5 relativ zur Arretierhülse 14 verdrehgesichert. Die Arretierhülse 14 ist verdrehgesichert und axial verschiebbar in der Injektionsvorrichtung, insbesondere relativ zu dem Gehäuse 12 und der Kupplungshülse 5 gelagert.

Während eines Ausschüttvorganges wird die Gewindehülse 13 durch Betätigung des Betätigungselements 15 in distale Richtung verschoben. Dabei drückt die Gewindehülse 13 auf das Lager 29, das in diesem Beispiel als Kugellager ausgebildet ist, jedoch auch als einfaches Gleitlager ausgebildet sein kann, wobei das Lager 29 auf die Arretierhülse 14 drückt, diese somit für einen Ausschüttvorgang in distale Richtung verschiebt und während eines Ausschüttvorgangs in distaler Position hält. Das Kupplungsglied 14 befindet sich somit distal der Abragungen der Kupplungshülse 5 für die Kupplung K1. Dadurch ist die Kupplung K1 für die Dauer des Ausschüttvorganges ausgekuppelt.

Beim Betätigen des Betätigungselements 15 verhalten sich die Kupplungen K1, K2 und K3 folgendermaßen: Durch Drücken des auf dem Kupplungsglied 10 und/oder Antriebswelle 7 sitzenden Druckknopfs 15 werden das Kupplungsglied 10 zusammen mit dem Druckknopf 15 und die Antriebswelle 7 in distale Richtung verschoben. Hierdurch kuppelt die Kupplung K2 ein, so dass die Antriebswelle 7 mit der Kupplungshülse 5 verdrehgesichert wird. Anschließend kuppelt die Kupplung K1 durch Verschiebung der Arretierhülse 14, auf welche die mit der Antriebswelle 7 verbundene Gewindehülse 13 über das axial verschiebbare Lager 29 drückt, aus. Die Kupplungen K1 und K2 können alternativ auch in umgekehrter Reihenfolge geschaltet werden.

Nach dem Einkuppeln von K2 und dem Auskuppeln von K1 kuppelt auch die Kupplung K3 durch Verschieben des Kupplungsglieds 10 relativ zum Dosierknopf 9 aus. Das Kupplungsglied 10, welches mit der Antriebswelle 7 verbunden ist, kann sich nach dem Auskuppeln der Kupplung K3 relativ zu dem Gehäuse 12 drehen. Die in der Antriebsfeder 3 während des Aufdosierens gespeicherte Energie oder Kraft kann auf die Antriebswelle 7 übertragen werden. Somit liegt an der Antriebswelle 7 ein Drehmoment an, welches mittels der eingekuppelten Kupplung K2 auf die Kupplungshülse 5 übertragen wird, welche sich zusammen mit der Antriebswelle 7 dreht und diese Drehbewegung auf das verdrehsicher mit der Kupplungshülse 5 gekoppelte Abtriebsglied 2 überträgt. Das in diesem Beispiel als Gewindestange ausgestaltete Abtriebsglied 2 setzt die Drehbewegung aufgrund des Gewindeeingriffs 2a, 6a mit dem Eingriffsglied 6, 26 in eine axiale Bewegung in distale Richtung um, so dass der am distalen Ende der Gewindestange 2 vorgesehene Flansch 1, der ebenfalls zum Abtriebsglied zugerechnet werden kann, in distale Richtung der Injektionsvorrichtung bewegt wird.

Da sich bei der Produktausschüttung die Gewindehülse 13 in entgegen gesetzte Richtung wie beim Aufdosieren dreht, dreht sich die Anzeigetrommel 4 ebenfalls in entgegen gesetzte Richtung wie beim Aufdosieren.

Im Normalfall, d.h. in dem Fall, bei dem die voreingestellte Produktdosis vollständig ausgeschüttet wird, verläuft der Ausschüttvorgang und insbesondere die Verschiebung des Abtriebsglieds 2 in distale Richtung so lange, bis der oben erwähnte in Umfangsrichtung wirkende Anschlag der Anzeigetrommel 4 anschlägt. Dies geschieht bevorzugt dann, wenn sich der durch das Fenster 12d ablesbare Wert auf 0 heruntergedreht hat.

In dem Fall, in dem der Benutzer der Vorrichtung das Beätigungselement 15 während der Produktausschüttung loslässt, kuppeln die Kupplungen in umgekehrter Reihenfolge, wie sie bei der Betätigung aus- bzw. eingekuppelt haben. Die Produktausschüttung wird unterbrochen, wobei durch das Fenster 12d der Wert ablesbar ist, der noch auszuschütten wäre, dass die voreingestellte Dosis komplett ausgeschüttet sein würde. Die Produktausschüttung kann dadurch fortgesetzt werden, dass das Betätigungselement 15 erneut gedrückt wird, wobei die Ausschüttung durch Loslassen des Betätigungselements 15 wieder gestoppt werden kann oder gewartet werden kann, bis die Produktausschüttung vollständig erfolgt ist.

Für den Fall, dass sich im Produktbehältnis weniger Produkt befindet als maximale Dosis auf der Anzeigetrommel angegeben ist, weist die in diesem Beispiel gezeigte Injektionsvorrichtung eine zusätzliche Einrichtung zur Begrenzung der letztmalig einstellbaren maximalen Dosis auf, um zu verhindern, dass eine größere Produktdosis eingestellt werden kann als sich Produkt in dem Produktbehältnis befindet. Hierzu ist ein Koppelglied 30 vorgesehen, das auch als Läufer bezeichnet werden kann. Der Läufer 30 umgreift die Kupplungshülse 5 zumindest teilweise und ist in solch einem Eingriff mit der Kupplungshülse 5, dass der Läufer 30 relativ zu der Kupplungshülse 5 drehfest und axial verschiebbar ist. Der Läufer 30 greift ferner mit einem an seinem äußeren Umfang gebildeten Gewinde in ein Innengewinde der Gewindehülse 13 ein. Diese Anordnung bewirkt, dass bei einer relativen Drehung zwischen Gewindehülse 13 und Kupplungshülse 5 eine Axialbewegung des Läufers 30 stattfindet, wobei bei keiner relativen Drehung der Läufer 30 keine Axialbewegung ausführt. Beim Einstellen einer Produktdosis wird die Gewindehülse 13 relativ zu der Kupplungshülse 5 verdreht, so dass der Läufer 30 in proximale Richtung wandert. Beim Ausschütten hingegen, findet keine Relativbewegung zwischen Kupplungshülse 5 und Gewindehülse 13 aufgrund des Kupplungseingriffs der Kupplung K2 statt. Die Läufer führt dann keine Bewegung aus. Nach mehrmaligem Dosieren und Produktausschütten gerät der Läufer 30 in einen axialen Anschlag mit der Antriebswelle 7, wodurch eine weitere Dosiserhöhung nicht mehr möglich ist, auch dann nicht mehr, wenn die Anzeige 4, 12d eigentlich mehr zuließe.

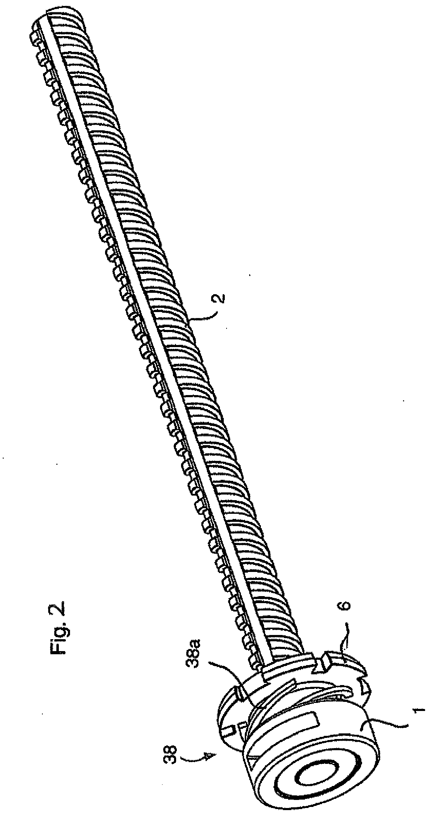

Der Verwender kann das Produktbehältnis 27 gegen ein neues austauschen. Hierzu entfernt er den Produktbehältnishalter 16 durch Drehung relativ zu dem Gehäuse 12 von der Antriebseinheit. Bei der Bewegung aus der befestigten Position in die unbefestigte Position des Produktbehältnisses 27, insbesondere beim Entriegeln des Bajonettverschlusses wird das Eingriffsglied 6, 26 zusammen mit dem Abtriebsglied 2 und der Kupplungshülse 5 relativ zu dem Gehäuse 12 und dem Kupplungsglied 14 in distale Richtung verschoben, wodurch die Kupplung K1 gelöst wird. Die für die Kupplung K1 vorgesehenen radial nach außen weisenden Abragungen der Kupplungshülse 5 befinden sich nun distal des Kupplungsglieds 14. Auf das Abtriebsglied 2, insbesondere dessen Flansch kann nun eine relativ geringe, in proximale Richtung wirkende Kraft aufgebracht werden, wodurch sich das Abtriebsglied 2 in die Antriebseinheit schraubt. Das Gewinde des Abtriebsglieds 2 ist nicht selbsthemmend. Beim Zurückschrauben des Abtriebsglieds 2 wird die Kupplungshülse 5 relativ zu der Gewindehülse 13 und zwar entgegensetzt wie bei der Produktausschüttung verdreht, wodurch der Läufer 30 wieder axial verschoben wird in distale Richtung. Das Zurückschrauben kann zumindest über einen Teil des Gesamtwegs gegen die Kraft eines Federglieds erfolgen, das z.B. versucht, das Abtriebsglied in distale Richtung zu verschieben. Das Federglied kann z.B. zwischen dem Abtriebsglied 2 und der Antriebswelle 7 wirken bzw. angeordnet sein. Weitere vorteilhafte Federglieder werden insbesondere zu Figur 2 weiter unten beschrieben. Allgemein bevorzugt wird, dass die Kraft eines solchen Federglieds geringer ist als die für eine Wechselwirkung über den Kolben auf das Produkt von dem Abtriebsglied 2 erforderliche Kraft.

Ferner wird beim Entfernen des Produktbehältnisses 27 das Halteglied 25, das dazu dient, das Produktbehältnis 27 in der Produktbehältnishalterung 16 zu fixieren, von der Feder 19 in distale Richtung verschoben bis es in einen Anschlag mit dem Eingriffsglied 6, 26 gerät. Dieser Anschlag verhindert, dass die Feder 19 sich bei entferntem Produktbehältnis 27 nicht vollständig entspannen kann. Dies ist vorteilhaft, da die Feder 19 auch bei einem entfernten Produktbehältnis 27 genügend Kraft aufbringen sollte, die Kupplung K3 in einem Kupplungseingriff zu halten, wodurch auch das Betätigungselement 15 in seiner proximalsten Stellung verharrt.

Gemäß einem weiteren Aspekt kann ein gefederter Flansch realisiert werden, wie z.B. in Fig. 2 gezeigt.

Nach einem Wechsel des bevorzugt als Ampulle oder Karpule ausgestalteten Produktbehältnisses 27 wird der Anwender, wie in der Bedienungsanleitung beschrieben, zum so genannten Entlüften oder Primen aufgefordert. Dies ist erforderlich, da sich zum einen Luft in dem Produktbehältnis 27 befindet und zum anderen das Abtriebsglied 2 zuvor vollständig in die Antriebseinheit hineingeschoben wurde und durch den unterschiedlichen Füllstand des Produktbehältnisses 27 etwas Spiel zwischen dem Kolben 28 und dem Flansch 1 entstanden ist.

Figur 2 zeigt ein Abtriebsglied 2 mit einer seinem vorderen oder distalen Ende befestigten Flansch 1, der unverschiebbar mit der Gewindestange verbunden ist. Zwischen dem Flansch 1 und dem in Figur 2 gezeigten Gewindeeinsatz 6 ist ein Federglied 38 vorgesehen, welches z.B. durch schräg abstehende Federarme 38a realisiert werden kann. Diese Federarme 38a können am Flansch 1 oder/und am Gewindeeinsatz 6, befestigt sein. Ebenso könnte auch ein Elastomer am Flansch 1 oder/und am Gewindeeinsatz 6 angespritzt werden. Nach dem Einsetzen eines neuen Produktbehältnisses 27 kann es zu einem Spiel zwischen dem Flansch 1 und dem Kolben 28 kommen, was insbesondere auf unterschiedliche Füllstände bei vollen Produktbehältnissen 27 zurückzuführen ist, die eine gewisse Toleranz aufweisen.

Nach dem Einschieben des mit der Gewindestange 2 verbundenen Flansches 1 liegt der Flansch 1 gemäß der in Figur 1 gezeigten Ausführungsform unmittelbar an dem Gewindeeinsatz 6 an.

Gemäß der in Figur 2 gezeigten Ausführungsform wird der Flansch 1 jedoch durch das mindestens eine Federglied 38 vom Gewindeeinsatz 6 um eine vorgegebene Distanz in distale Richtung weggedrückt. Dies ermöglicht es, dass bei einem eingesetzten Produktbehältnis 27 oder beim Einsetzen des Produktbehältnisses 27 der Flansch 1 immer auf der proximalen Seite des Kolbens 28 zum Anliegen kommt, selbst wenn der Kolben 28 bei verschiedenen Produktbehältnissen bedingt durch Fertigungstoleranzen unterschiedlich weit in das Produktbehältnis 27 eingeschoben ist.

Herkömmliche Maßnahmen zur Beseitigung des Spiels zwischen Flansch 1 und Kolben 28 sind daher nicht mehr zwingend erforderlich und können z.B. sogar entfallen.

Wie aus Figur 1 zu erkennen ist, weist die Injektionsvorrichtung, insbesondere die Antriebseinheit eine Bremse 17, 18 auf, die ein sich drehendes Teil, in diesem Beispiel das Übertragungselement 7, K2, 5 oder/und die Antriebsbewegung abbremst. Bei herkömmlichen Injektionsvorrichtungen besteht bei Fehlanwendungen, z.B. dann, wenn kein Produktbehältnis eingelegt ist und dennoch eine Betätigung der Injektinosvorrichtung vorgenommen wird, die Gefahr von Überbelastung oder sogar einer Schädigung der Bauteile der Injektionsvorrichtung. Bei einem eingelegten Produktbehältnis 27 wird durch die Viskosität des Produkts bei der Produktausschüttung eine Dämpfung der auftretenden Kräfte und Bewegungen vorgenommen. Bei einem fehlenden Produktbehältnis fehlt ein solches Dämpfungsglied. Abhilfe schafft die Bremse 17, 18, die Überbelastungen vermeidet.

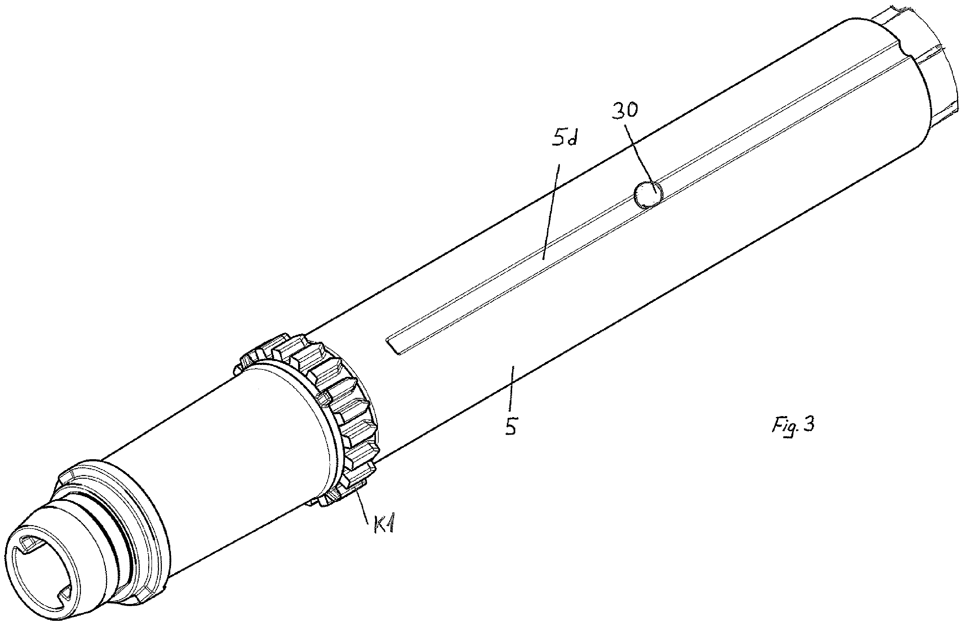

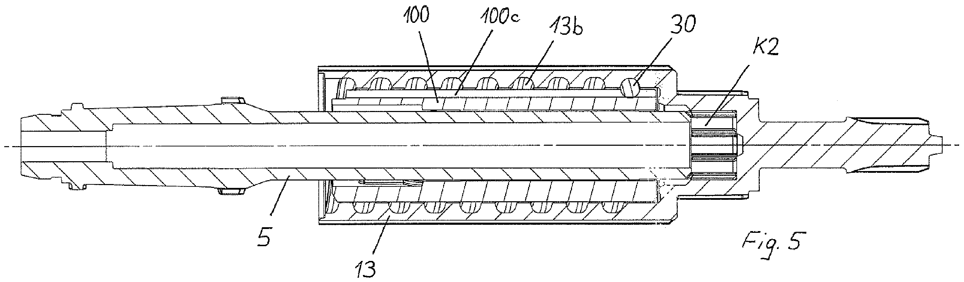

Die modifizierte Kupplungshülse 5 aus Figur 3, die wie die Kupplungshülse 5 aus Figur 1 als zweites Element dient, ist im Grunde so aufgebaut, wie die Kupplungshülse aus Figur 1. Insbesondere weist die Kupplungshülse 5 eine sich parallel zur Drehachse, um die die Kupplungshülse drehbar ist, erstreckende Führungsbahn 5d, insbesondere eine Längsnut auf. In die Führungsbahn greift das in diesem Beispiel als Kugel ausgestaltete Koppelglied 30 ein. Die Führungsbahn 5d ist im Querschnitt abgerundet, insbesondere mit einem Radius, der in etwa dem Kugelradius entspricht. Durch die Führungsbahn 5d wird die Kugel 30 parallel zur Längsachse der Kupplungshülse 5 geführt. Statt einer Kugel könnte auch ein anderer Rotationskörper von der Führungsbahn 5d geführt werden, wie zum Beispiel ein Zylinder. Die Kugel 5d greift ferner in die als Gewinde ausgestaltete Führungsbahn 13b der als erstes Element dienenden Gewindehülse 13 ein (z.B. Figur 1 oder 5). Die Führungsbahn 13b kann ebenfalls im Querschnitt so abgerundet sein, wie die Führungsbahn 5d.

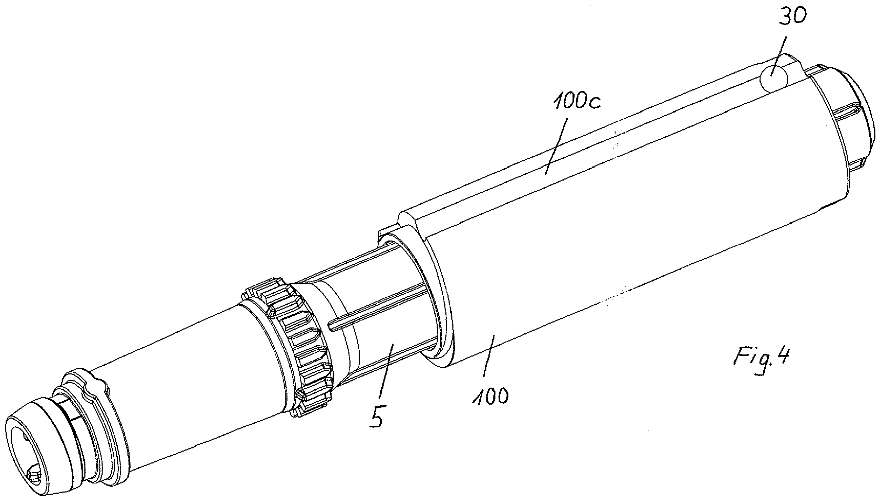

Die in den Figuren 4 und 5 gezeigte Modifikation entspricht im Prinzip der Ausführung aus Figur 3, wobei die Führungsbahn 100c für die Kugel 30 nicht von der Kupplungshülse 5 sondern von einer Zwischenhülse 100 gebildet wird, nämlich an deren äußeren Umfang. Die Zwischenhülse 100 ist mit und relativ zu der Kupplungshülse 5 drehfest und axialverschiebbar verbunden, wie in diesem Beispiel mit Längsführungen, die am Außenumfang der Kupplungshülse 5 gebildet sind und in die am Innenumfang der Zwischenhülse 100 gebildete Abragungen eingreifen.

Der Vorteil der Zwischenhülse 100 in allen hierin gezeigten Modifikationen ist, dass sie einerseits auf die Kupplungshülse 5 aus Figur 1 aufgesetzt werden kann und andererseits bei einem Austausch des Produktbehältnisses die Zwischenhülse 100 keine Längsbewegung ausführen braucht, da die Kupplungshülse 5 relativ zur Zwischenhülse 100 längsbewegbar ist.

Bei den Ausführungsformen aus den Figuren 3 bis 5 wird bei der Dosiseinstellung die Gewindehülse 13 relativ zu der Kupplungshülse 5, dem Koppelglied 30 und ggf. der Zwischenhülse 100 verdreht, wobei das Koppelglied 30 relativ zu der Kupplungshülse 5 oder der Zwischenhülse 100 drehfest ist und eine Längsbewegung entlang der Führungsbahn 5d oder 100c ausführt. "Drehfest" bezieht sich auf die Drehung der Kugel 30 relativ zu der Längsachse der Kupplungshülse 5 oder der Zwischenhülse 100 und nicht auf die Drehbarkeit der Kugel 30 um ihren Kugelmittelpunkt. Die Kugel 30 gerät in ihrer Stoppposition in den Anschlag mit dem Ende der Führungsbahn 13b und der Flanke der Längsführung 5d oder 100c, wodurch eine Dosiserhöhung verhindert wird.

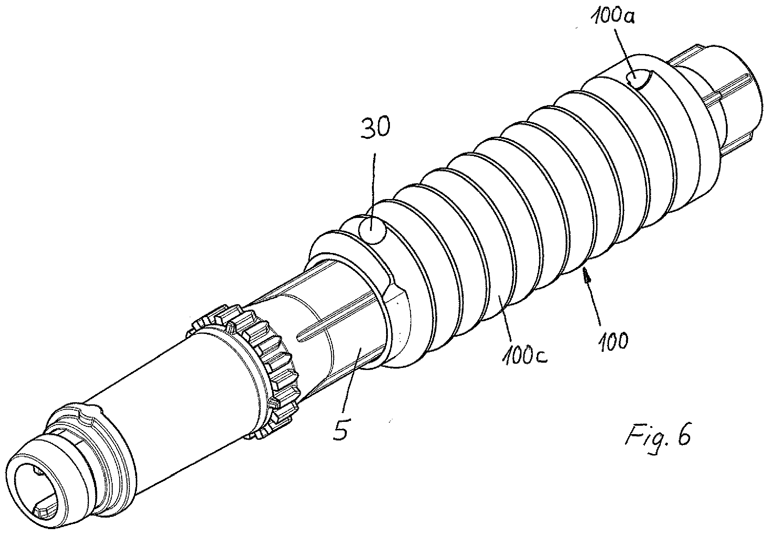

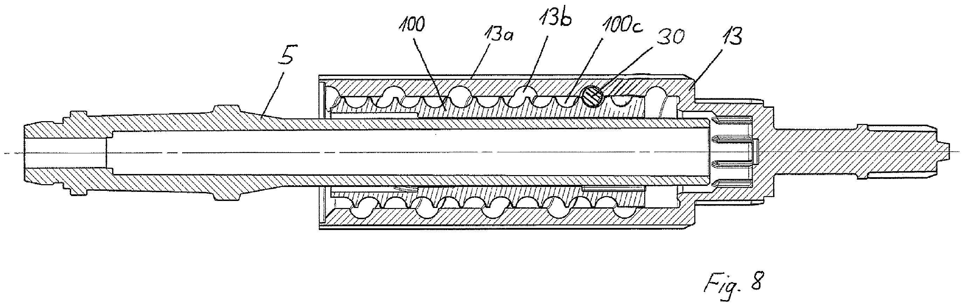

Die in den Figuren 6 bis 8 gezeigte Modifikation weist eine Zwischenhülse 100 auf, die wie die Zwischenhülse 100 aus den Figuren 4 und 5 mit der Kupplungshülse 5 verbunden ist und die statt einer sich parallel zur Längsachse erstreckende Führungsbahn 100c eine sich gewinde- oder wendelförmig entlang der Längsachse erstreckende Führungsbahn 100c aufweist. Die gewinde- oder wendelförmige Führungsbahn 100c weist eine geringere Steigung als die gewinde- oder wendelförmige Führungsbahn 13b der Gewindehülse 13 auf. Die Führungsbahn 100c weist einen der Führungsbahn 13b entgegen gesetzten Drehsinn auf. Wie insbesondere aus Figur 8 erkennbar ist, ist die Kugel 30 dort angeordnet, wo die Führungsbahnen 13b und 100c sich überkreuzen und eine Einfassung für die Kugel 30 bilden. An der Stelle, wo sich die Führungsbahnen 13b und 100c überkreuzen, bilden sie eine in der Projektion rautenförmige Einfassung, in der die Kugel mit einem Spiel geführt ist.

Bei der Drehung der Gewindehülse 13 relativ zu der Zwischenhülse 100 wird die Kugel 30 mit der Gewindehülse 13 kontinuierlich mitgedreht, aber mit einer geringeren Winkelgeschwindigkeit als sich die Gewindehülse 13 dreht. Durch die untersetzte Bewegung der Kugel 30 ist der Mechanismus platzsparend.

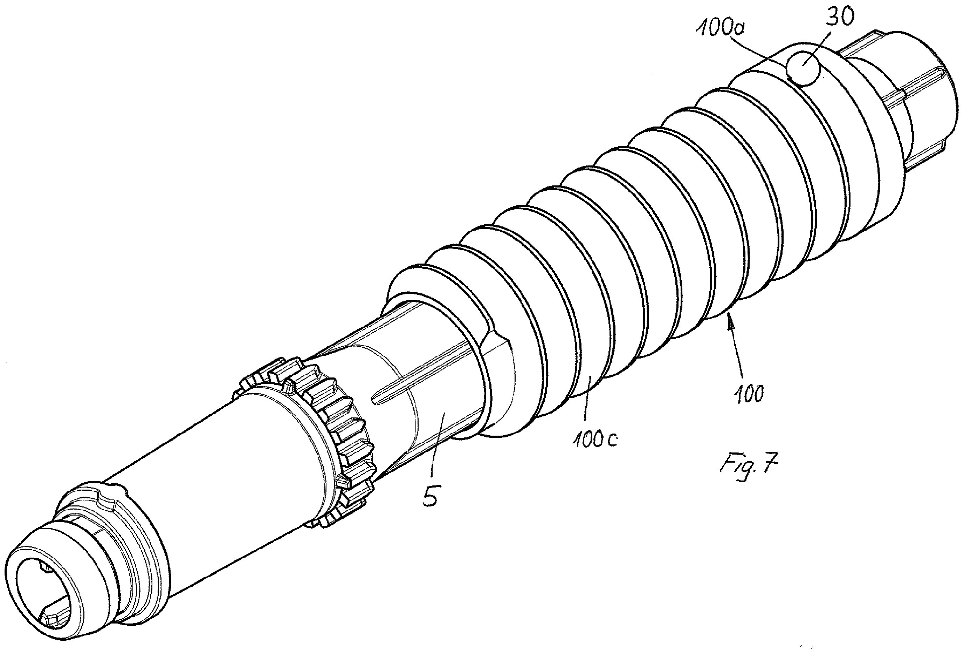

Bei jeder Dosiserhöhung wird die Kugel 30, die sich bei einem vollen Produktbehältnis in der in Figur 6 gezeigten Position befindet, um ein zur eingestellten Dosis entsprechendes Stück in Richtung Stoppanschlag 100a, der vom Ende des Gewindes 100c gebildet wird, bewegt. In Figur 7 befindet sich die Kugel in ihrer Stoppposition, d.h. im Anschlag mit dem Stoppanschlag 100a. Das Ende der Führungsbahn 13c befindet sich in der in Figur 7 gezeigten Position der Kugel ebenfalls in einem Anschlag mit der Kugel 30, nämlich dem Anschlag 100a gegenüber liegend, wobei in Figur 7 die Gewindehülse 13 zu Darstellungszwecken weggelassen wurde.

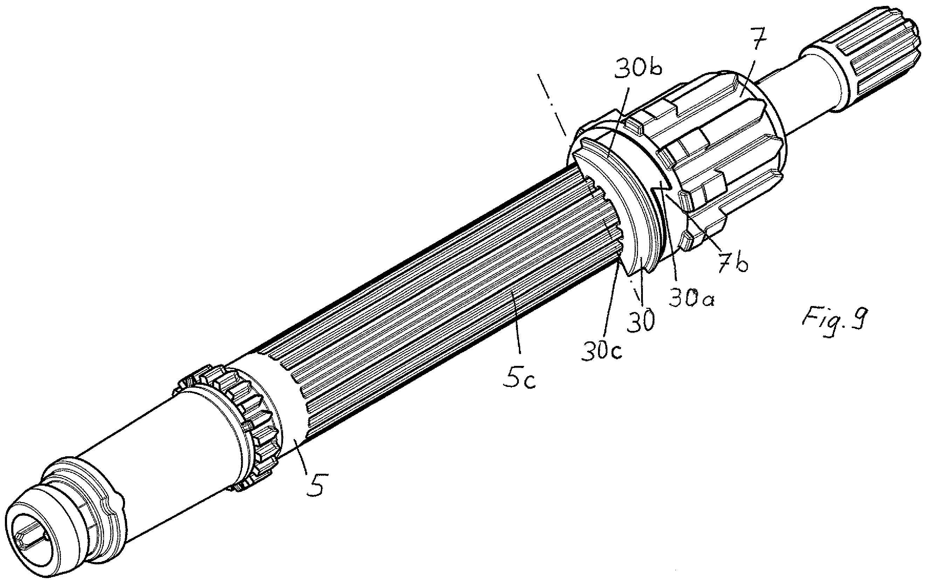

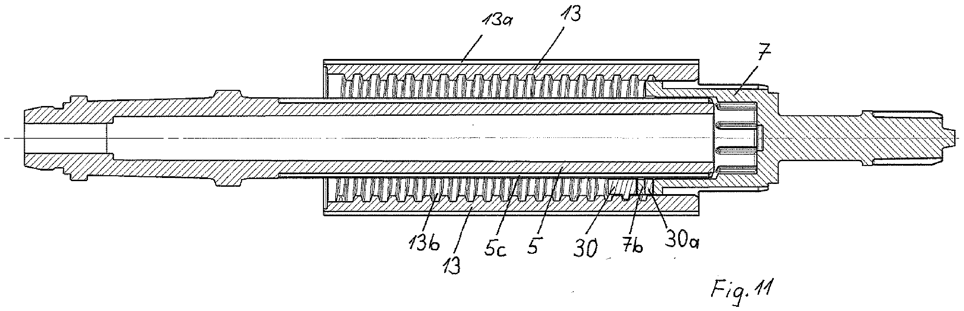

In den Figuren 9 bis 11 wird eine Modifikation für die Vorrichtung aus Figur 1 mit einem als Segment 30 ausgestalteten Koppelglied gezeigt, wobei das Segment 30 mit Abragungen 30c in Längsführungen 5c der Kupplungshülse 5 eingreift. Das Segment 30 ist relativ zur Kupplungshülse 5 drehfest und längsbewegbar. Das Segment 30 weist an seinem Außenumfang einen Gewindeabschnitt 30b auf, der in das Gewinde 13b eingreift. Das Segment 30 wird bei einer Dosiserhöhung in Richtung Stoppposition bewegt, die in Figur 9 gezeigt ist und in der ein Anschlag 7b in einen Eingriff mit dem Anschlag 30a ist, wodurch eine Drehung der Antriebswelle 7 und der damit verbundenen Gewindehülse 13 relativ zu der Kupplungshülse 5 und dem Segment 30 verhindert wird.

Die Anschläge 7b, 30a weisen quer zur Längsrichtung angeordnete Anschlagflächen auf, durch deren Form das Segment 30 zu der Antriebswelle 7 hingezogen wird. Durch die Form der korrespondierend geneigten Anschlagflächen ist der in Umfangsrichtung wirkende Drehanschlag sehr stabil.

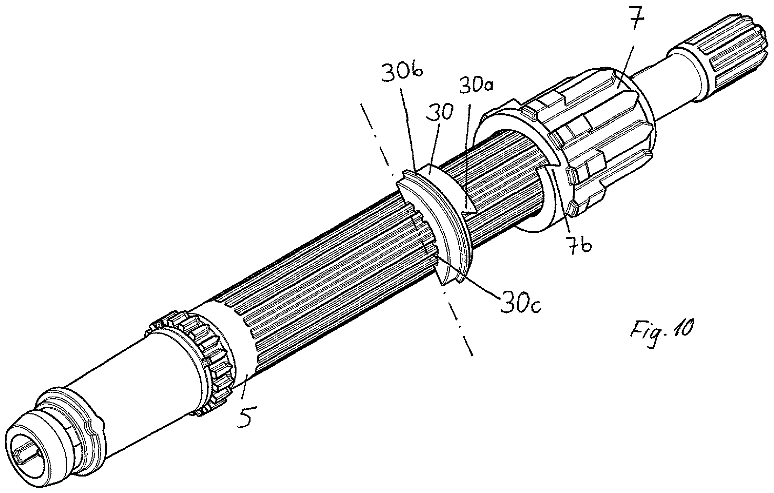

Das Segment weist in Umfangsrichtung zwei Enden auf, die in den Figuren 9 und 10 jeweils mit einer als Strichpunktlinie gezeichneten Verbindungsgeraden verbunden sind. Diese gedachte Verbindungsgerade schneidet die Kupplungshülse 5 jedoch nicht die in der Kupplungshülse 5 aufgenommene Kolbenstange 2, sondern tangiert oder passiert diese. Es wird nicht der gesamte Ringspalt zwischen Gewindehülse 13 und Kupplungshülse 5 ausgefüllt, wodurch das Segment 30 platzsparend ist.

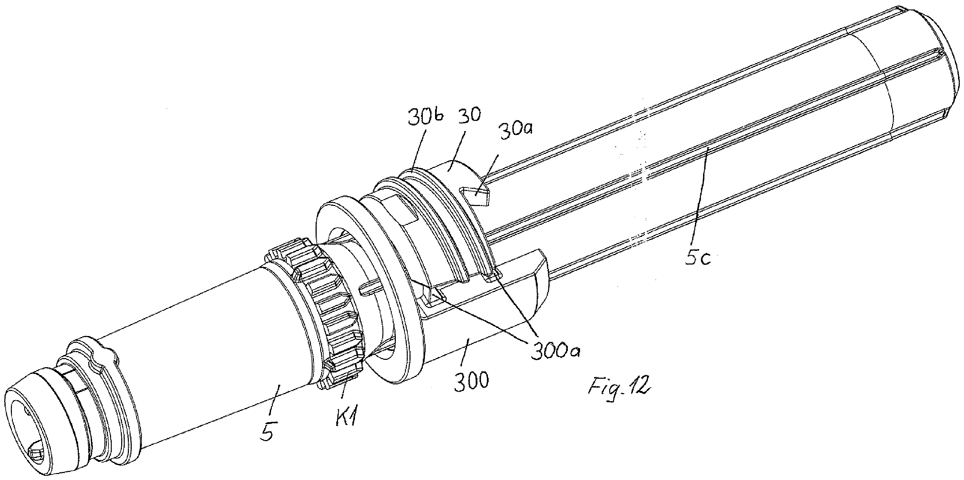

Das in Figur 12 gezeigte Segment 30 ist eine vorteilhafte Weiterbildung des Segments aus den Figuren 9 bis 11 und prinzipiell auch so aufgebaut. Allerdings ist das Segment 30 mit mehreren Sollbruchstellen 300a stoffschlüssig an einem Basiselement 300 gebildet. Bei der Montage das einteilige, aus dem Segment 30 und dem Basiselement 300 gebildete Teil auf die Kupplungshülse 5 aufgesteckt, wobei das Basiselement 300 wie das Segment 30 über die Längsführungen 5c verdrehgesichert ist. Allerdings ist das Basiselement im Gegensatz zum Segment 30 axialfest relativ zu der Kupplungshülse 5. Bei einer Dosiseinstellung, insbesondere Dosiserhöhung wird das Segment 30 in Richtung Stoppposition relativ zu dem Basiselement 300 axialverschoben, wodurch die Sollbruchstellen 300a zerbrechen. Das Basiselement 300 ermöglicht vorteilhaft eine einfache Montage des Segments 30. Das Basiselement 300 bildet einen Hülsenabschnitt, von dem ein Teil das Segment 30 ist. Der am Basiselement 300 verbleibende Teil des Hülsenabschnitts kann ein Stützglied bilden, der die Kupplungshülse 5 gegen eine Bewegung quer zur Längsachse abstützt.

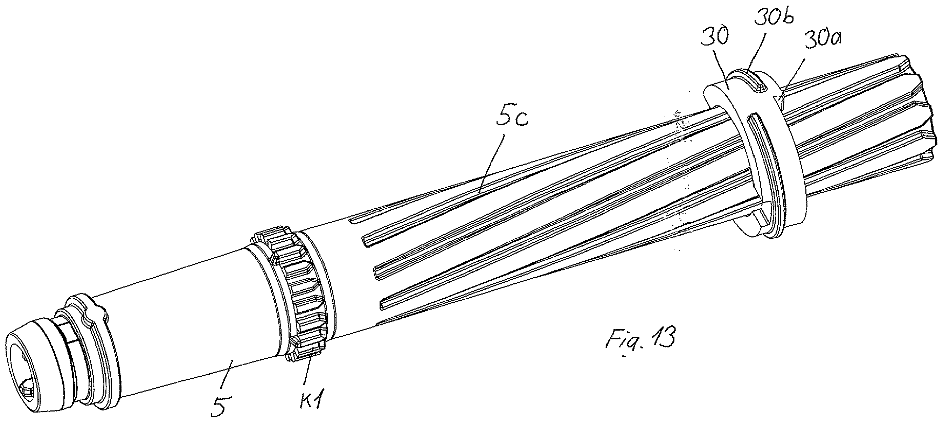

Figur 13 zeigt ein Koppelglied in der Gestalt einer Mutter 30, die die Kupplungshülse 5 vollständig umgibt, d.h. einen Durchgang bildet. Statt der Mutter 30 könnte beispielsweise ein Segment vorgesehen sein, da die Mutter abgesehen von der Tatsache, dass sie einen geschlossenen Ring bildet, wie das Segment aus den Figuren 9 bis 11 aufgebaut ist. Die Kupplungshülse aus Figur 13 weist jedoch eine gewindeförmige Führungsbahn 5c auf, deren Drehsinn dem des Gewindeabschnitts 13 entgegengesetzt ist und die eine größere Steigung aufweist als der Gewindeabschnitt 30b, der in das Gewinde 13b der Gewindehülse 13 eingreift. Durch die Anordnung wird die Mutter 30 bei der Dosiseinstellung mit der Gewindehülse kontinuierlich mitgedreht, wobei die Mutter 30 eine geringere Drehwinkelgeschwindigkeit ausführt als die Gewindemutter 13. Durch diese Untersetzung ist der Mechanismus platzsparend.

Die Gewindehülse 5 aus Figur 14 weist eine sich in proximale Richtung breiter werdende Führungsbahn 5c auf und kann z.B. mit einem mutterförmigen Koppelglied verwendet werden, wie z.B. dem aus Figur 13. Die sich aufweitende Führungbahn 5c erleichtert das Entformen der Kupplungshülse 5 aus einem Spritzgusswerkzeug. Die insbesondere nutförmige Führungsbahn 5c wird auf beiden Seiten von jeweils einer Flanke begrenzt, wobei wenigstens eine Flanke sich wendelförmig über den Umfang erstreckt. Die andere Flanke kann dies ebenfalls oder in etwa parallel zur Längsachse der Kupplungshülse 5 angeordnet sein. Hierdurch werden Vorteile der Modifikationen aus den Figuren 13 und 14 vereint, so dass die Kupplungshülse 5 einfach entformbar ist und die Stoppmutter 30, die bei einer Dosiserhöhung an der sich wendelförmig erstreckenden Flanke anliegt, eine kontinuierlich und untersetzte Drehbewegung ausführt, wenn die Kupplungshülse 5 gedreht wird.

Bei der Ausführungsform aus Figur 14 braucht sich bei einer Dosiskorrektur oder zumindest einem Teil der Dosiskorrektur die Stoppmutter 30 nicht in der umgekehrten Bewegungsabfolge wie beim Aufdosieren bewegen, da sich der Abstand zwischen der wendelförmigen Flanke und der parallel zur Längsachse der Kupplungshülse verlaufenden Flanke mit zunehmenden Weg in die proximale Richtung vergrößert. Dadurch entsteht mit zunehmendem Weg in proximale Richtung ein sich vergrößernder Spalt zwischen wenigstens einer den Flanken und dem Teil des Koppelglieds 30, der in die Führungsbahn 5c eingreift.

Figur 15 zeigt ein mehrteiliges, insbesondere zweiteiliges Koppelglied, das ein Führungsteil 30m und ein relativ dazu drehbares und axialfestes Anschlagteil 30n aufweist. Das Führungsteil ist an dem zweiten Element längsgeführt und greift in das Gewinde des ersten Elements. Das Anschlagteil 30n weist einen in Umfangsrichtung wirkenden Anschlag 30a auf. Zwischen Führungsteil 30m und Anschlagteil 30n ist eine Ratsche 301 angeordnet, die fühl- und hörbare Klicks erzeugt, wenn das Führungsteil 30m relativ zu dem Anschlagteil 30n verdreht wird, nämlich dann, wenn der Anschlag 30 a in einen entsprechenden Gegenanschlag ist, wie er z.B. Figur 9 mit dem Bezugszeichen 7b gekennzeichnet ist. Hierdurch wird das Erreichen der Stoppposition dem Verwender bei den letzten im Produktbehältnis enthaltenen Dosen angekündigt. Führungsteil 30m und Anschlagteil 30n können optional mit einer Feder (nicht dargestellt) rotatorisch gegeneinander vorgespannt sein.

## Patentansprüche

1. Injektionsvorrichtung, umfassend:
a) ein Dosiseinstellelement (9; 10; 7; 4) und ein damit gekoppeltes erstes Element (13), ,
b) eine Kupplung, die bei einer Dosiseinstellung in einem ersten Kupplungszustand ist und bei einer Dosisausschüttung in einem zweiten Kupplungszustand ist, wobei die Kupplung so mit dem ersten Element (13) und mit einem anderen, zweiten Element (2; 5; 100) verbunden ist, dass im ersten Kupplungszustand das erste Element (13) relativ zum zweiten Element (2; 5; 100) drehen kann und im zweiten Kupplungszustand das erste Element (13) relativ zum zweiten Element (2; 5; 100) drehfest ist, wobei das erste Element (13) und das zweite Element (2; 5; 100) über ein Koppelglied (30) gekoppelt sind, und
c) einen Stoppanschlag (7b; 100a), wobei bei einer Dosiseinstellung das Koppelglied (30) eine Bewegung zu einer Stoppposition hin ausführt, wobei das Koppelglied (30) in der Stoppposition im Anschlag mit dem Stoppanschlag (7b; 100a) die Einstellung einer Dosis verhindert,
**dadurch gekennzeichnet, dass**
d) wenigstens eines aus erstem Element (13) und zweitem Element (2; 5; 100) eine gewindeförmige Führungsbahn (13b; 100c) aufweist und das andere aus erstem Element (13) und zweitem Element (2; 5; 100) eine gewindeförmige oder eine sich parallel zur Längsachse der Injektionsvorrichtung erstreckende Führungsbahn (5d; 13e; 100c) aufweist,
e) wobei die Führungsbahn (13b; 100c) des ersten Elements (13) eine erste Teileinfassung und die Führungsbahn (5d) des zweiten Elements (2; 5; 100) eine zweite Teileinfassung bildet, die zusammen eine Einfassung für das Koppelglied (30) bilden, die das Koppelglied (30) zumindest so einfasst, dass ein Schwerpunkt des Koppelglieds (30) dort verbleibt, wo sich die Führungsbahnen (5d; 13b; 100c) überkreuzen.

2. Injektionsvorrichtung nach Anspruch 1, wobei das Koppelglied (30) eine Kugel oder ein Segment ist.

3. Injektionsvorrichtung nach einem der vorhergehenden Ansprüche, wobei das Koppelglied (30) in eine Führungsbahn, insbesondere Innengewinde des ersten Elements (13) und in eine Führungsbahn, insbesondere Aussengewinde des zweiten Elements (2; 5; 100) eingreift.

4. Injektionsvorrichtung nach einem der vorhergehenden Ansprüche, wobei das Koppelglied (30) so mit dem ersten Element (13) und dem zweiten Element (2; 5; 100) gekoppelt ist, dass das Koppelglied (30) bei einer Bewegung des ersten Elements (13) relativ zum zweiten Element (2; 5; 100) relativ zu wenigstens einem, vorzugsweise beiden aus dem ersten Element (13) und dem zweiten Element (2; 5; 100) drehbar ist oder gedreht wird.

5. Injektionsvorrichtung nach einem der vorhergehenden Ansprüche, wobei das Koppelglied (30) bei einer Drehung des ersten Elements (13) relativ zu dem zweiten Element (2; 5; 100) um einen Drehwinkel mitgedreht wird, der grösser oder vorzugsweise kleiner ist als der Drehwinkel des ersten Elements (13).

6. Injektionsvorrichtung nach einem der Ansprüche 1, 2 und 4, wobei das Koppelglied (30) in eine Längsführung (5d; 100c), die an einem aus erstem Element (13) und zweiten Element (2; 5; 100) gebildet ist, und in eine gewindeförmige Führungsbahn (5c; 100c; 13b), das an dem anderen aus erstem Element (13) und zweitem Element (2; 25; 100) gebildet ist, eingreift.

7. Injektionsvorrichtung nach einem der vorhergehenden Ansprüche, wobei das erste drehbare Element (13) drehfest mit einer Dosisanzeigehülse (4) oder/und einer Drehfeder (3) gekoppelt ist.

8. Injektionsvorrichtung nach einem der vorhergehenden Ansprüche, wobei das zweite drehbare Element (5; 100) drehfest mit einem Abtriebsglied (2), insbesondere einer Kolbenstange gekoppelt ist, wobei das Abtriebsglied (2) insbesondere für eine Produktausschüttung in Ausschüttrichtung schraubbar ist.

9. Injektionsvorrichtung nach einem der vorhergehenden Ansprüche, wobei das erste drehbare Element (13) und/oder das zweite drehbare Element (2; 5; 100) hülsenförmig sind.

10. Injektionsvorrichtung nach einem der vorhergehenden Ansprüche, wobei wenigstens eine der Führungsbahnen (5d; 13b; 100c) im Querschnitt konkav und/oder abgerundet und/oder in etwa kreisförmig ist.

11. Injektionsvorrichtung nach einem der vorhergehenden Ansprüche, wobei wenigstens eine der Führungsbahnen (5d; 13b; 100c) einen in Axialrichtung oder in Umfangrichtung wirkenden Anschlag (100a) für das Koppelglied (30) bildet.

12. Injektionsvorrichtung nach einem der vorhergehenden Ansprüche, wobei die Drehung des Koppelglieds (30) relativ zu dem ersten Element (13) oder zu dem zweiten Element (2; 5; 100) kleiner ist als eine volle Umdrehung, wenn das Koppelglied (30) von der Position, in der es am weitesten von der Stoppposition entfernt ist, in die Stoppposition bewegt wird.

## Claims

1. An injection device including:
a) a dose setting element (9; 10; 7; 4) and a first element (13) coupled thereto,
b) a coupling which in a dose setting situation is in a first coupling state and in a dose dispensing situation is in a second coupling state, wherein the coupling is connected to the first element (13) and another second element (2; 5; 100) in such a way that in the first coupling state the first element (13) can rotate relative to the second element (2; 5; 100) and in the second coupling state the first element (13) is non-rotatable relative to the second element (2; 5; 100), wherein the first element (13) and the second element (2; 5; 100) are coupled by way of a coupling member (30), and
c) a stop abutment (7b; 100a) wherein in a dose setting situation the coupling member (30) performs a movement towards a stop position, wherein the coupling member (30) in the stop position abutting against the stop abutment (7b; 100a) prevents setting of a dose,
**characterised in that**
d) at least one of the first element (13) and the second element (2; 5; 100) has a thread-form guide path (13b; 100c) and the other of the first element (13) and the second element (2; 5; 100) has a guide path (5d; 13e; 100c) which is of thread form or extends parallel to the longitudinal axis of the injection device,
e) wherein the guide path (13b; 100c) of the first element (13) forms a first partial enclosure and the guide path (5d) of the second element (2; 5; 100) forms a second partial enclosure which together form an enclosure for the coupling member (30) which encloses the coupling member (30) at least in such a way that a centre of gravity of the coupling member (30) remains where the guide paths (5d; 13b; 100c) intersect.

2. An injection device according to claim 1 wherein the coupling member (30) is a ball or a segment.

3. An injection device according to one of the preceding claims wherein the coupling member (30) engages into a guide path, in particular a female thread of the first element (13), and a guide path, in particular a male thread of the second element (2; 5; 100).

4. An injection device according to one of the preceding claims wherein the coupling member (30) is coupled to the first element (13) and the second element (2; 5; 100) in such a way that upon a movement of the first element (13) relative to the second element (2; 5; 100) the coupling member is rotatable or is rotated relative to at least one and preferably both of the first element (13) and the second element (2; 5; 100).

5. An injection device according to one of the preceding claims wherein upon a rotation of the first element (13) relative to the second element (2; 5; 100) the coupling member (30) is also rotated through a rotational angle which is greater than or preferably less than the rotational angle of the first element (13).

6. An injection device according to one of claims 1, 2 and 4 wherein the coupling member (30) engages into a longitudinal guide (5d; 100c) formed from one of the first element (13) and the second element (2; 5; 100) and into a thread-form guide path (5c; 100c; 13b) formed on the other of the first element (13) and the second element (2; 5; 100).

7. An injection device according to one of the preceding claims wherein the first rotatable element (13) is non-rotatably coupled to a dose display sleeve (4) and/or a torsion spring (3).

8. An injection device according to one of the preceding claims wherein the second rotatable element (5; 100) is non-rotatably connected to a drive output member (2), in particular a piston rod, wherein the drive output member (2) can be screwed for product dispensing in the dispensing direction.

9. An injection device according to one of the preceding claims wherein the first rotatable element (13) and/or the second rotatable element (2; 5; 100) are sleeve-shaped.

10. An injection device according to one of the preceding claims wherein at least one of the guide paths (5d; 13b; 100c) is concave in cross-section and/or rounded and/or approximately circular.

11. An injection device according to one of the preceding claims wherein at least one of the guide paths (5d; 13b; 100c) forms an abutment (100a) acting in the axial direction or in the peripheral direction for the coupling member (30).

12. An injection device according to one of the preceding claims wherein the rotation of the coupling member (30) relative to the first element (13) or the second element (2; 5; 100) is less than a full revolution when the coupling member (30) is moved from the position in which it is furthest away from the stop position into the stop position.

## Revendications

1. Dispositif d'injection comprenant :
a) un élément de réglage de dose (9 ; 10 ; 7 ; 4) et un premier élément (13) couplé à celui-ci,
b) un couplage, qui est dans le cas d'un réglage de dose dans un premier état de couplage et est dans le cas d'une décharge de dose dans un deuxième état de couplage, dans lequel le couplage est relié de telle sorte au premier élément (13) et à un autre deuxième élément (2 ; 5 ; 100) que dans le premier état de couplage, le premier élément (13) peut tourner par rapport au deuxième élément (2 ; 5 ; 100) et dans le deuxième état de couplage, le premier élément (13) est solidaire en rotation par rapport au deuxième élément (2 ; 5 ; 100), dans lequel le premier élément (13) et le deuxième élément (2 ; 5 ; 100) sont accouplés par l'intermédiaire d'un organe d'accouplement (30), et
c) une butée d'arrêt (7b ; 100a), dans lequel dans le cas d'un réglage de dose, l'organe d'accouplement (30) exécute un déplacement en direction d'une position d'arrêt, dans lequel l'organe d'accouplement (30) empêche dans la position d'arrêt en butée avec la butée d'arrêt (7b ; 100a) le réglage d'une dose,
**caractérisé en ce que**
d) au moins un parmi le premier élément (13) et le deuxième élément (2 ; 5 ; 100) présente une voie de guidage (13b ; 100c) en forme de filetage et l'autre parmi le premier élément (13) et le deuxième élément (2 ; 5 ; 100) présente une voie de guidage (5d ; 13e ; 100c) en forme de filetage ou une voie de guidage s'étendant de manière parallèle par rapport à l'axe longitudinal du dispositif d'injection,
e) dans lequel la voie de guidage (13b ; 100c) du premier élément (13) forme un premier encadrement partiel et la voie de guidage (5d) du deuxième élément (2 ; 5 ; 100) forme un deuxième encadrement partiel, qui forment conjointement un encadrement pour l'organe d'accouplement (30), qui encadre l'organe d'accouplement (30) au moins de telle sorte qu'un centre de gravité de l'organe d'accouplement (30) reste là où les voies de guidage (5d ; 13b ; 100c) s'entrecroisent.

2. Dispositif d'injection selon la revendication 1, dans lequel l'organe d'accouplement (30) est une sphère ou un segment.

3. Dispositif d'injection selon l'une quelconque des revendications précédentes, dans lequel l'organe d'accouplement (30) vient en prise avec une voie de guidage, en particulier un filetage intérieur du premier élément (13) et avec une voie de guidage, en particulier un filetage extérieur du deuxième élément (2 ; 5 ; 100).

4. Dispositif d'injection selon l'une quelconque des revendications précédentes, dans lequel l'organe d'accouplement (30) est accouplé de telle sorte avec le premier élément (13) et le deuxième élément (2 ; 5 ; 100) que l'organe d'accouplement (30) peut tourner ou est tourné dans le cas d'un déplacement du premier élément (13) par rapport au deuxième élément (2 ; 5 ; 100) par rapport à au moins un, de préférence les deux parmi le premier élément (13) et le deuxième élément (2 ; 5 ; 100).

5. Dispositif d'injection selon l'une quelconque des revendications précédentes, dans lequel l'organe d'accouplement (30) est entraîné en rotation dans le cas d'une rotation du premier élément (13) par rapport au deuxième élément (2 ; 5 ; 100) autour d'un angle de rotation, qui est plus grand ou de préférence plus petit que l'angle de rotation du premier élément (13).

6. Dispositif d'injection selon l'une quelconque des revendications 1, 2 et 4, dans lequel l'organe d'accouplement (30) vient en prise dans un système de guidage longitudinal (5d ; 100c), qui est formé au niveau d'un parmi le premier élément (13) et le deuxième élément (2 ; 5 ; 100), et avec une voie de guidage (5c ; 100c ; 13b) en forme de filetage, qui est formée au niveau de l'autre parmi un premier élément (13) et un deuxième élément (2 ; 5 ; 100).

7. Dispositif d'injection selon l'une quelconque des revendications précédentes, dans lequel le premier élément rotatif (13) est accouplé de manière solidaire en rotation à une douille d'affichage de dose (4) et/ou à un ressort de torsion (3).

8. Dispositif d'injection selon l'une quelconque des revendications précédentes, dans lequel le deuxième élément (5 ; 100) rotatif est accouplé de manière solidaire en rotation à un organe de sortie (2), en particulier à une tige de piston, dans lequel l'organe de sortie (2) peut être vissé en particulier pour une décharge de produit dans une direction de décharge.

9. Dispositif d'injection selon l'une quelconque des revendications précédentes, dans lequel le premier élément (13) rotatif et/ou le deuxième élément (2 ; 5 ; 100) rotatif sont en forme de douille.

10. Dispositif d'injection selon l'une quelconque des revendications précédentes, dans lequel au moins une des voies de guidage (5d ; 13b ; 100c) est de section transversale concave et/ou arrondie et/ou de forme à peu près circulaire.

11. Dispositif d'injection selon l'une quelconque des revendications précédentes, dans lequel au moins une des voies de guidage (5d ; 13b ; 100c) forme une butée (100a), agissant dans la direction axiale ou dans la direction périphérique, pour l'organe d'accouplement (30).

12. Dispositif d'injection selon l'une quelconque des revendications précédentes, dans lequel la rotation de l'organe d'accouplement (30) par rapport au premier élément (13) ou par rapport au deuxième élément (2 ; 5 ; 100) est inférieure à un tour complet, quand l'organe d'accouplement (30) est déplacé depuis la position, dans laquelle il est le plus éloigné de la position d'arrêt, dans la position d'arrêt.
